# EUROPEAN PATENT SPECIFICATION

(11) **EP 2 077 830 B1**
(45) Date of publication and mention of the grant of the patent: **07.11.2012**
(21) Application number: 07843370.3
(22) Date of filing: 27.09.2007
(51) Int. Cl.: A61K 31/135, A61K 31/167, A61K 31/575, A61P 3/04

(54) **METHODS, COMPOSITIONS, AND FORMULATIONS FOR THE TREATMENT OF THYROID EYE DISEASE**
PROZESSE, ZUSAMMENSETZUNGEN U. FORMULIERUNGEN ZUR BEHANDLUNG DER SCHILDDRÜSEN AUGENKRANKHEIT
PROCÉDÉS, COMPOSITIONS, ET FORMULATIONS POUR LE TRAITEMENT DE LA MALADIE DE L'OEIL LIÉE À LA THYROÏDE

(30) Priority: 17.10.2006 US 852221 P; 29.01.2007 US 898009 P; 20.03.2007 US 919011 P
(43) Date of publication of application: 15.07.2009
(73) Proprietor: Lithera, Inc., California 92122 (US)
(72) Inventor: DOBAK, John Daniel, La Jolla, CA 90237 (US)
(74) Representative: Cole, William Gwyn
(86) International application number: PCT/US2007/079740
(87) International publication number: WO 2008/048770

(56) References cited:
- EP-A1- 1 153 614
- WO-A-2004/028545
- WO-A1-98/48810
- WO-A1-03/033000
- WO-A2-01/19373
- WO-A2-2004/103057
- WO-A2-2007/011743
- US-A1- 2005 113 456
- US-B1- 6 316 443
- ADCOCK IAN M ET AL: "Molecular interactions between glucocorticoids and long-acting beta2-agonists" JOURNAL OF ALLERGY AND CLINICAL IMMUNOLOGY, MOSBY, INC, US, vol. 110, no. 6, suppl, 1 December 2002 (2002-12-01), pages S261-S268, XP009129953 ISSN: 0091-6749 [retrieved on 2002-12-31]
- SECO A J ET AL: "Acute and chronic treatment with glucocorticosteroids, modifying the beta2-adrenergic response of the Guinea pig trachea" LUNG,, vol. 173, no. 5, 1 March 1995 (1995-03-01) , pages 321-328, XP009129940
- ALLERGY 1998,, vol. 53, no. 42 Suppl, 1 January 1998 (1998-01-01), pages 7-13, XP002570036
- MIRKIN G: "Albuterol for weight control" INTERNET CITATION 13 July 1994 (1994-07-13), pages 1-2, XP002560518 Retrieved from the Internet: URL:http://www.drmirkin.com/archive/6247.h tml> [retrieved on 2009-12-14]
- M:H: Beers et al.: "The Merck Manual" 1999, Merck and CO , NJ, USA , XP002570537 , page 706-707 * See Exophthalmosis or proptosis *
- FRIES P.D.: 'Thyroid dysfunction: Managing the ocular complications of Graves' GERIATRICS vol. 47, no. 2, 1992, pages 58 - 70, XP008105124
- LANGLEY R.W. ET AL.: 'Perioperative management of the thyrotoxic patient' ENDOCRINOLOGY AND METABOLISM CLINICS OF NORTH AMERICA vol. 32, no. 2, 2003, pages 519 - 534, XP008105125
- HALL K. ET AL.: 'Intravenous methylpredinisolone in the treatment of Graves' ophthalmopathy' BMJ vol. 297, no. 6663, 1988, pages 1574 - 1578, XP008105136
- GITTOES N.J.L. ET AL.: 'Hyperthyroidism. Current Treatment Guidelines' DRUGS vol. 55, no. 4, 1998, pages 543 - 553, XP008105126

## Description

### BACKGROUND OF THE INVENTION

Graves' disease is a common disorder with an incidence in women of 1/1000 population/year. In addition to hyperthyroidism, 25-50% of individuals with Graves' disease develop clinical involvement of the eyes, i.e., thyroid eye disease. Graves' ophthalmopathy (GO) is a typical form of thyroid eye disease. While some patients with GO experience only suffer mild ocular discomfort, 3-5% suffer from intense pain and inflammation with double vision or even loss of vision.

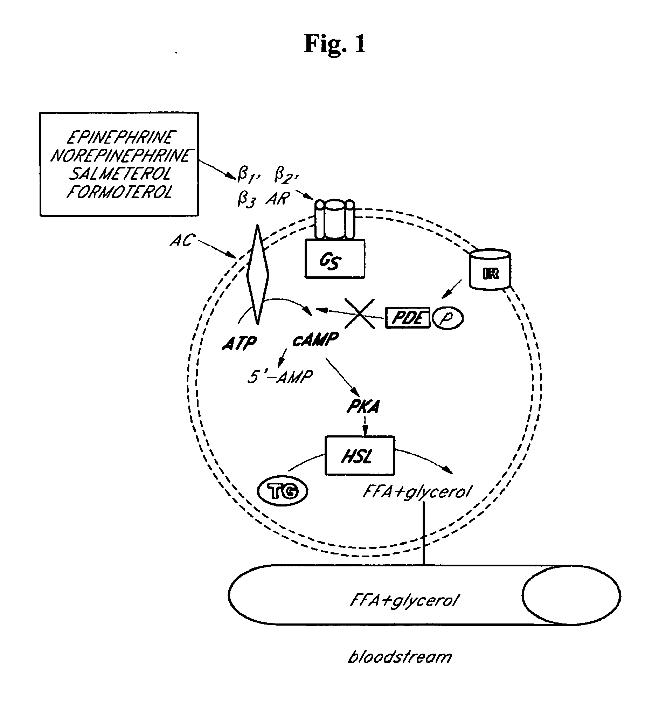

The clinical symptoms and signs of GO can be explained mechanically by the increase in tissue volume evident within the bony orbit. The expanded orbital tissues cause forward displacement of the globe and impairment of venous and lymphatic outflow from the orbit. These changes, combined with the local production of cytokines and other mediators of inflammation, result in proptosis, periorbital edema, conjunctival erythema and chemosis (Figure 1).

WO 2004/103057 discloses pharmaceutical compositions, methods for increasing the rate of apoptosis in adipose tissue cells and methods of reducing adipose tissue mass in a host. WO 98/48810 discloses a method for improving health, survival and muscle growth rate of animals, while reducing carcass fat and improving feed efficiency by administering an optically pure eutomer of an adrenergic beta-2 agonist. US 2005/113456 discloses a method which utilises an optically pure eutomer of salbutamol for reducing body fat and/or body weight in mammals and birds.

WO 2004/028545 discloses a combination of a long-acting beta-2 agonist and a glucocorticosteroid in the treatment of fibrotic diseases. WO 03/033000 discloses pharmaceutical combinations comprising salmeterol and fluticasone propionate for the treatment of asthma.

### SUMMARY OF THE INVENTION

Described herein are compositions, formulations, methods, and systems for treating thyroid eye disease by contacting a targeted fat deposit in the eye with a composition comprising long acting beta-2 adrenergic receptor agonist and a compound that reduces beta adrenergic receptor desensitization in the target tissue to the long acting beta-2 adrenergic receptor agonist. Embodiments of the composition are administered, for example, by retrobulbar (behind the eye) injection, and/or transocularly. The glucocorticosteroid has the added effect of reducing inflammatory cells and release of inflammatory cytokines present in the orbit and the adipose tissue of the orbit.

Accordingly, in one aspect provided herein is a therapeutically effective amount of at least one beta adrenergic agonist and a therapeutically effective amount of at least one compound for reducing beta-adrenergic receptor desensitization for use in reducing orbital fat accumulation in a patient in need thereof (e.g., a subject suffering from thyroid eye disease). In some embodiments, the patient in need of the just-described treatment is suffering from Graves' Ophthalmopathy. In some embodiments, the patient in need of treatment is suffering from enlargement of extraocular muscles. In some embodiments, the patient is suffering from proptosis.

In some embodiments, administration of the at least one beta adrenergic agonist or the at least one compound for reducing desensitization is parenteral, oral, intraocular, intraorbital, intraconal, ophthalmic, retrobulbar, periorbital, topical, intramuscular, transdermal, sublingual, intranasal, or respiratory.

In some embodiments, the at least one compound for reducing beta-adrenergic receptor desensitization is administered before (e.g., about 3 days to about 7 days before) the at least one beta adrenergic agonist. In some embodiments, the at least one compound is administered by an intraocular, intraorbital, ophthalmic, periorbital, retrobulbar, or intraconal route of administration. In some embodiments, the at least one compound is administered in the form of a crystalline microparticle suspension.

In some embodiments, the at least one compound is orally administered and the at least one beta adrenergic agonist is ophthalmically administered. In the at least one ophthalmically administered beta adrenergic agonist is administered in a crystalline microparticle formulation.

In some embodiments, the at least one beta adrenergic agonist to be administered to the patient comprises a long acting beta adrenergic agonist. In some embodiments, the at least one compound is a glucocorticosteroid and the long acting beta adrenergic agonist is salmeterol, formoterol, or a combination thereof. In some embodiments, the beta adrenergic agonist to be administered is a beta adrenergic agonist that is selective for the beta-2 adrenergic receptor. In some embodiments, the at least one beta adrenergic agonist comprises salmeterol, formoterol, or any combination thereof. In some embodiments, the at least one beta adrenergic agonist comprises salmeterol and the therapeutically effective amount of salmeterol is about 0.01 µg/day to about 100 µg/day (e.g., about 1 µg/day to about 100 µg/day, about 10 µg/day to about 100 µg/day, or about 50 µg/day to about 100 µg/day) of salmeterol. In other embodiments, the at least one beta adrenergic agonist comprises formoterol and the therapeutically effective amount of formoterol is about 0.001 µg/day to about 50 µg/day (e.g., 0.01 µg/day to about 1.0 µg/day, about 0.1 µg/day to about 10 µg/day, about 1 µg/day to about 20 µg/day, or about 5 µg/day to about 40 µg/day).

The at least one compound for reducing beta adrenergic receptor desensitization is glucocorticosteroid. In some embodiments, the at least one compound for reducing beta adrenergic receptor desensitization comprises dexamethasone, prednisolone, methylprednisolone, fluticasone propionate, budesonide, ketotifen, or any combination thereof.

In some embodiments, the patient is administered a therapeutically effective amount of an immunosuppressant agent by an intraocular, intraorbital, ophthalmic, periorbital, retrobulbar, or intraconal route before administration of the at least one beta adrenergic agonist and the at least one compound for reducing beta adrenergic receptor desensitization. In some embodiments, the immunosuppressant agent is administered in the form of a crystalline microparticle suspension.

In another aspect provided herein is a composition comprising a therapeutically effective amount of at least one beta adrenergic agonist for use in treating proptosis.

In some embodiments, the at least one beta adrenergic agonist comprises a long-acting beta adrenergic agonist. In some embodiments, the at least one beta adrenergic agonist comprises a beta adrenergic agonist that is selective for the beta-2 adrenergic receptor. In some embodiments, the at least one beta adrenergic agonist comprises salmeterol, formoterol, bambuterol, eformoterol, isoproterenol, albuterol, or fenoterol. In some embodiments, the composition comprises a mixture of at least one long-acting beta adrenergic agonist and at least one short-acting beta adrenergic agonist. In some embodiments, the composition also comprises a therapeutically effective amount of hyaluronidase.

In some embodiments, the composition comprises salmeterol and the patient is administered a therapeutically effective amount of salmeterol from about 0.01 µg/day to about 100 µg/day. In other embodiments, the composition comprises formoterol and the patient is administered a therapeutically effective amount of formoterol from about 0.001 µg/day to about 50 µg/day.

In some embodiments, administration of the composition is parenteral, oral, intraocular, intraorbital, periorbital, ophthalmic, retrobulbar, intraconal, topical, intramuscular, transdermal, sublingual, intranasal, or respiratory.

Described herein is a method for reducing orbital fat accumulation in patient in need of treatment by administering to the patient a therapeutically effective amount of one or more adrenergic receptor pathway stimulating compounds and a therapeutically effective amount of at least one compound for reducing beta adrenergic receptor desensitization. The one or more adrenergic receptor pathway-stimulating compounds may comprise a catecholamine, an alpha adrenergic antagonist, forskolin, aminophylline, or analogs thereof.

In yet another aspect provided herein is an ophthalmic pharmaceutical composition comprising an ophthalmically acceptable excipient and a therapeutically effective amount of methylprednisolone acetate or fluticasone propionate in the form of a crystalline microparticle suspension. In some embodiments, the ophthalmic pharmaceutical composition further comprises solubilized methylprednisolone acetate or solubilized fluticasone propionate. In some embodiments, the ophthalmic pharmaceutical composition further comprises a therapeutically effective amount of at least one long acting beta-2 agonist in the form of a crystalline microparticle suspension.

In another aspect provided herein is ophthalmic pharmaceutical composition comprising an ophthalmically acceptable excipient and a therapeutically effective amount of at least one long acting beta-2 agonist in the form of a crystalline microparticle suspension. In some embodiments, the at least one long acting beta-2 agonist comprises salmeterol or formoterol. In some embodiments, the ophthalmic pharmaceutical composition further comprises a therapeutically effective amount of at least one solubilized long acting beta-2 agonist. In some embodiments, the ophthalmic pharmaceutical composition further comprises a therapeutically effective amount f of at least one compound for reducing beta adrenergic receptor desensitization in the form of a crystalline microparticle suspension.

In a further aspect provided herein is at least one beta adrenergic agonist and at least one compound for reducing beta adrenergic receptor desensitization for use in treating a disease involving orbital fat accumulation.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

**FIG. 1** is a schematic illustration of adipocyte lipolysis

**FIG. 2** is a bar graph illustrating the dose-dependent induction of lipolysis in cultured adipocytes by the long acting beta-2 agonist Formoterol after a three hour incubation.

**FIG. 3** is a bar graph illustrating the dose-dependent induction of lipolysis in cultured adipocytes by the long acting beta-2 agonist Salmeterol after a three hour incubation.

**FIG. 4** is a bar graph illustrating the dose-dependent induction of lipolysis in cultured adipocytes by the glucocorticosteroid Budesonide after a short incubation period (three hours), and the suppression of lipolysis after longer incubation periods (18 hours).

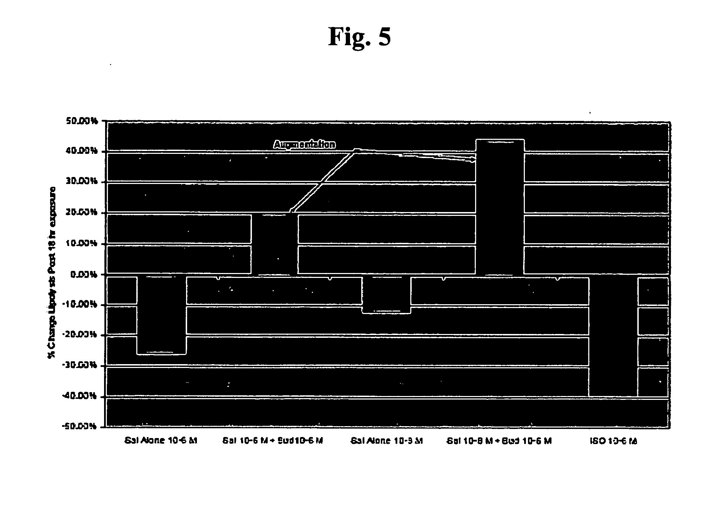

**FIG. 5** is a bar graph illustrating the dose-dependent suppression of lipolysis in cultured adipocytes by the long acting beta-2 agonist Salmeterol given alone for 18 hours, and the dose-dependent induction of lipolysis by Salmeterol after 18 hours when given in combination with the glucocorticosteroid Budesonide.

**FIG. 6** is a bar graph illustrating average within-animal differences in epididymal fat pad mass (left fat pad versus right fat pad) in fat pads injected with vehicle solution (2% PEG), Formoterol alone, or Formoterol plus Budesonide over a three day treatment period.

**FIG. 7** is a bar graph illustrating the dose-dependent reduction of fat pad mass for two different dose combinations of the beta-2 agonist Formoterol and the glucocorticosteroid Budesonide over a three day treatment period.

### DETAILED DESCRIPTION OF THE PREFERRED EMBODIMENT

Computerized tomographic scans show that the majority of patients with GO have enlargement of both the orbital fat and the extraocular muscles, while others appear to have only adipose tissue or extraocular muscle involvement. The extraocular muscles cells themselves are intact in early, active disease, suggesting that they are not themselves the targets of autoimmune attack. Rather, the enlargement of the extraocular muscle bodies results from an accumulation of hydrophilic mucopolysaccharides, including especially hyaluronan, within the perimysial connective tissues. In later stage disease, the resolving inflammatory process within the muscles may leave them fibrotic and misaligned.

The increase in the volume of the adipose/connective tissues within the orbit appears to contribute more significantly to the overall expanded orbital tissue volume than does the extraocular muscle enlargement. Computerized tomographic studies show that proptosis measurements in these patients are most closely correlated with the volume of the fat compartment. This expanded adipose tissue volume appears to result both from hyaluronan accumulation with attendant edema, and from the emergence of a population of newly differentiated fat cells within these tissues.

Histologic examination of orbital tissues in GO reveals that the characteristic changes result primarily from hyaluronan accumulation with edema, expansion of the fat compartment, and infiltration of the tissues by T lymphocytes. Orbital fat cells in this condition appear to be differentiated, though smaller than other fat cells of the body (e.g. subcutaneous or omental). Orbital fat cells of GO express higher levels of PPAR-gamma, adiponectin, and leptin mRNA transcripts. In addition, orbital fat cells may less 11-beta-hydroxysteroid dehydrogenase type I, an enzyme involved in the conversion of cortisone to the active form cortisol.

Studies using cells obtained from these tissues have shown that the orbital fibroblast is an orbital cell that may participate in these diverse cellular processes. These cells are particularly sensitive to stimulation by cytokines and other immune mediators, responding by increasing CD40 expression, synthesizing large quantities of hyaluronan, and secreting inflammatory cytokines. In addition, the preadipocyte subpopulation of fibroblasts is capable of differentiating into mature adipose cells that exhibit high levels of TSHR. Fibroblasts have also been shown to display IGF-1R. When bound by IgG from Graves' patients, these receptors initiate downstream signaling that results in RANTES and IL-16 production and leads to local lymphocytic infiltration. The relative site-specificity of orbital involvement in Graves' disease may be explained both by the relative sensitivity of these fibroblasts to immune mediators, and by the unique anatomical features of these sites that appear to predispose them to compression of low-pressure lymphatic and venous channels.

Adipose tissue is the primary energy storage tissue of the body. Fat cells, or adipocytes, store this energy in the form of triglycerides. Triglycerides are mobilized from fat stores to provide caloric energy to the body through hormonal induction of triglyceride hydrolysis. This process releases free or non-esterified fatty acids and glycerol into the blood for use by other body tissues. The breakdown of triglycerides from fat store is referred to as lipolysis. Growth of new adipocytes also occurs, which is referred to as adipogenesis.

Catecholamines are the primary regulators of adipose tissue through the adrenergic receptors. Adipose tissue has beta-1, 2, and 3 adrenergic receptors and alpha-2 adrenergic receptors. Binding of beta agonists to beta receptors in adipose tissue can result in adipocyte lipolysis, while binding of alpha receptor agonists can inhibit lipolysis. Beta receptor activation can also inhibit adipogenesis. In humans, the beta-2 receptor are often the most abundant on fat cell surfaces and the primary mediator of beta receptor-stimulated lipolysis. Stimulation of lipolysis by beta agonists is mediated by adenylate cyclase and increased formation of cyclic adenosine monophosphate (cyclic AMP, cAMP). Alpha 2 receptors reduce lipolysis in mature fat cells. Alpha-2 adrenergic receptors may be involved in the proliferation of pre-adipocytes. Glucocorticosteroids may have a permissive effect on adipose tissue and increase responses of adipocytes, such as lipolysis, to catecholamine stimulation. This permissive action may be due to up-regulation of beta-adrenergic receptors and other components involved in secondary intracellular messengers.

The treatment of GO would necessarily target reduce the volume of the expanded orbital fat tissue. Hence, a formulation to reduce adipocyte volume through lipolysis may prove useful for this condition. Further, a reduction in the inflammatory process and inflammatory cells in the orbit that may be related to the adipose tissue expansion may further reduce orbital tissue volumes. Further, treatment of the hyaluraonan accumulation in the orbit may provide additional volume reduction and relief of the ophthalmopathy. Lastly, inhibition of adipogenesis may improve the condition.

Delivery of adrenergic active ingredients into the subcutaneous tissue, both beta agonists and alpha-2 antagonists, has been proposed and has been shown to result in regional fat loss and improved appearance of regional fat accumulations. For example, isoproterenol 11 and yohimbine 8 have been shown to reduce the thigh circumference in women. Because these lipolytic agents, especially the beta agonists, are short-acting and may be rapidly removed from the adipose tissue, the lipolysis is likely to have occurred for only a short time after the injection thereby reducing the potential magnitude of the effect despite the multiple injections. Additionally, long term exposure of adipocytes to beta agonists results in receptor desensitization and down regulation, and a loss of lipolytic activity. Means to reduce or prevent these effects on the receptor may also improve the therapy. Nonetheless, a strategy to treat the adipocyte in GO using adrenergic agents and glucocorticosteroids to induce lipolysis and inhibit adipogenesis may provide effective reduction of the tissue mass responsible for the clinical signs and symptoms.

Described herein are embodiments of pharmaceutical compositions, formulations, methods, and systems for treating thyroid eye disease (e.g., Grave's ophthalmopathy or "GO") by reducing the orbital tissue mass. Reducing orbital tissue mass may reduce proptosis, restore or prevent vision loss and diplopia, and reduce pain. This tissue mass reduction may be accomplished by producing at least one of the following including: reducing orbital fat mass, reducing inflammation (e.g. inflammatory cells and cytokines), and reducing glycosaminoglycan (GAG) accumulation. Fat mass reduction may be achieved through adrenergic system modulation. As used and/or cited herein, the term "modulation" is generally used in its usual sense, and more particularly to refer to adrenergic receptor agonism, adrenergic receptor antagonism, and/or changes in receptor signaling pathways. One example of a change in receptor signaling pathways includes an increase in cyclic AMP, for example as illustrated schematically in FIG. 1. In some embodiments, modulation refers to receptor upregulation or an increase in the number of adrenergic receptors, a decrease in receptor deactivation or sequestration, receptor activity changes (for example, an increase in activity), and/or changes in receptor affinity. Modulation of adrenergic receptors may be preferably produced with the use of a glucocorticosteroid or antihistamine, which will also work to reduce inflammation. Glycosaminoglycan accumulation can be reduced similarly with the use of the glucocorticosteroid and may be further reduced with the use of an enzyme to degrade hyaluronic acid, such as recombinant human hyaluronidase.

Reduction of orbital tissue fat mass is preferably achieve in a non-ablative manner, by initiating lipolysis, inhibiting adipogenesis, or reducing lipid accumulation. Ablative methods for reducing fat such as phosphatidyl choline or deoxycholate may be problematic for use behind the eye where non selective destruction of tissue may result in nerve or muscle damage or may cause scarring and fibrosis. Stimulating lipolysis, inibiting adipogenesis, and reducing lipid accumulation may be achieved through stimulation of beta adrenergic receptors. Stimulation of beta adrenergic receptors may also counter some of the cellular transcripts known to be upregulated in orbital fat cells in graves such as PPAR-gamma, adiponectin, and leptin. For example, stimulation of the beta adrenergic receptor may reduce PPAR-gamma and adiponectin expression in differentiated adipocytes. It is believed that some embodiments of sustained modulation of adrenergic receptors in adipose tissue result in some combination of sustained lipolysis, reduced lipid content of the adipocyte, reduced adipocyte cell size, reduced adipose tissue mass or fat accumulation, and/or reduced. Some embodiments provide selective reduction of orbital accumulations of adipose tissue and adipocytes, through sustained adrenergic modulation. Sustained adrenergic modulation results in sustained inhibition of fat cell proliferation (adipogenesis) in some embodiments.

Various embodiments of the disclosed pharmaceutical compositions comprise at least one selective beta-2 adrenergic receptor agonist (e.g., a long acting selective beta-2 agonist) in combination with at least one compound that reduces desensitization of beta-adrenergic receptors, e.g., desensitization of the target tissue to the beta-adrenergic receptor agonist(s), i.e., glucocorticosteroids , and also reduce inflammation. The term desensitization includes both short term desensitization (tachyphylaxis), as well as long term desensitization, as well as desensitization over other time periods. Beta-2 adrenergic receptor agonists are also referred to herein as "beta-2 agonists" and "beta-2 receptor agonists." Unless otherwise specified, references to beta-2 adrenergic receptor agonists also include their analogs, physiologically acceptable salts and/or solvates. Some embodiments of the composition comprise from about 100:1 to about 1:100 long-acting selective beta-2 agonist to glucocorticosteroid.

As discussed above, lipolytic activity, adipocyte proliferation inhibition, and reduction in lipid accumulation are believed to be mediated through modulation of adrenergic receptors in adipose tissue and/or on adipocytes. In some embodiments, the reduction therapy is enhanced through prolonged exposure or sustained activity of one or more adrenergic receptor agonists and/or receptor pathway stimulating compounds, for example, catecholamines, beta agonists, alpha antagonists, forskolin, aminophylline, analogs thereof, or combinations thereof.

Some embodiments provide sustained adrenergic modulation through the use of pharmaceutical compositions comprising one or more long-acting substantially selective beta-2 receptor agonists. Some embodiments of the sustained activity pharmaceutical composition comprise one or more suitable long-acting, selective beta-2 agonists, for example, salmeterol 1, formoterol 2, bambuterol 3, eformoterol physiologically acceptable salts or solvates thereof, or combinations thereof.

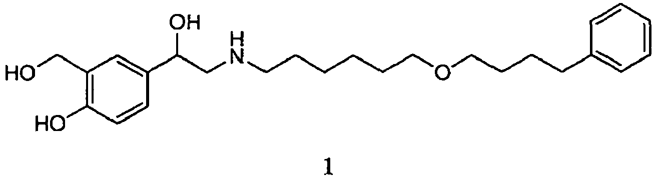

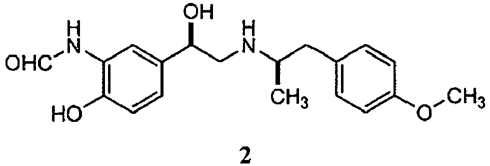

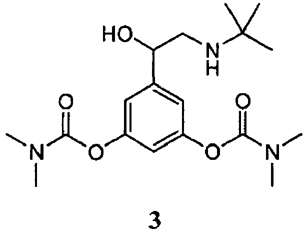

Sustained adrenergic modulation may not be observed with typical adrenergic compositions because the adrenergic compound is generally rapidly removed from the adipose tissue through the blood and/or lymph in part due to their hydrophilicity. Furthermore, long-term exposure of adipose tissue to adrenergic agents, particularly beta receptor agonists, is believed to result in receptor desensitization through receptor phosphorylation and sequestration. It is believed that these effects limit the ability of an adrenergic modulating composition to treat adipose tissue and result in tachyphylaxis, a condition in which the body experiences a rapidly decreasing response to the agonist following administration of the initial doses, to the desired lipolytic and anti-adipogenesis effect. Consequently, the treatment effect is short lived.

Short-acting beta-2 agonists often result in tachyphylaxis, as discussed above. However, because preferred embodiments of long-acting selective beta-2 agonists have substantially selective beta-2 receptor activity and high lipophilicity, the activities of long-acting beta-2 agonists continue for longer periods of time in adipose tissue compared with short-acting beta-2 agonists. Partial beta-2 receptor antagonist activity, which occurs with the use of salmeterol, may prevent some desensitization that can occur with continuous exposure of adipocytes to full adrenergic agonists. Further, salmeterol may not completely activate the arrestin signaling that leads to receptor internalization and degradation and leads to long term receptor down regulation. Compared with short-acting beta-2 agonists, lipolysis also occurs for a longer time after administration because long-acting selective beta-2 agonists have longer half lives. The combination of longer half-lives and activities may reduce the frequency of administration of the pharmaceutical compositions. Consequently, in some embodiments, daily administration or more than once daily administration of the composition is not required. Moreover, preferred embodiments of long-acting selective beta-2 agonists also exhibit greater selectivity for beta-2 receptors, permitting substantially similar therapeutic effects compound with short-acting beta-2 agonists at a lower dosage. Further the more selective beta-2 activity can limit cardiac side effects, which are often induced by beta-1 receptor stimulation in the heart.

As discussed above, lipolysis and/or inhibition of adipogenesis and lipid accumulation are stimulated by the beta-1, 2, or 3 receptor subtypes. Thus, agonists to one, two and/or all three receptors are capable of stimulating lipolysis and/or inhibition of adipogenesis. In humans, beta-2 receptor activity is believed to be more important for stimulating lipolysis, particularly in the presence of an anti-inflammatory steroid or glucocorticosteroid.

Long-acting selective beta-2 agonists, for example, salmeterol **1** (±2-(hydroxymethyl)-4-[1-hydroxy-2-[6-(4-phenylbutoxy)hexylamino]ethyl]-phenol, CAS Reg. No. 94749-08-3), and formoterol **2** (± *N*-[2-hydroxy-5-[1-hydroxy-2-[1-(4-methoxylphenyl)propan-2-ylamino]ethyl]-phenyl]methanamide, CAS Reg. No. 73573-87-2), are preferred in some embodiments. Some embodiments of the compositions comprise one or more long-acting selective beta-2 agonists as physiologically acceptable salts or solvates, for example, salmeterol xinafoate and/or formoterol fumarate. In many cases, salts and/or solvates of a beta-2 agonists will have the desired activity. Accordingly, unless otherwise specified, references to an active ingredient, for example, to salmeterol 1, formoterol **2**, isoproterenol **4**, albuterol **5**, fenoterol, and forskolin, include the compounds themselves as well as a physiologically acceptable analogs, salts, and/or solvates thereof, or combinations thereof.

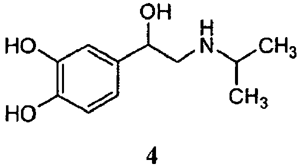

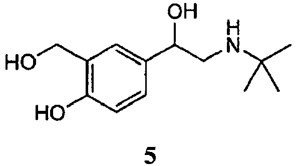

Some preferred long-acting beta agonists exhibit high intrinsic adenylate cyclase activity, which increase cAMP synthesis. For example, some embodiments comprise formoterol **2** as a long-acting beta-2 selective agonist, which exhibits some combination of higher potency, reduced systemic effects, high intrinsic activation of adenylate cyclase, and/or increases in cyclic AMP, a mediator of lipolysis.

In some preferred embodiments formoterol **2** is present as a physiologically acceptable salt and/or solvate thereof. Suitable physiologically acceptable salts of formoterol **2** include, for example, acid addition salts derived from inorganic and organic acids, such as the hydrochloride, hydrobromide, sulfate, phosphate, maleate, fumarate, tartrate, citrate, benzoate, 4-methoxybenzoate, 2-hydroxybenzoate, 4-hydroxybenzoate, **4**-chlorobenzoate, *p*-toluenesulphonate, methanesulphonate, ascorbate, salicylate, acetate succinate, lactate, glutarate, gluconate, tricarballylate, hydroxynaphthalenecarboxylate, oleate, combinations thereof, and the like. Preferred embodiments comprise formoterol **2** as its fumarate salt and/or as a dihydrate. Suitable tissue concentration of formoterol **2** for adipose tissue treatment include from about 1 pM to about 100 µM, more preferably from about 0.1 nM to about 10 uM, e.g., about 1 nM to about 1 µM, about 40 nM to about 3 µM, about 0.1 µM to about 1 µM, or any other tissue concentration of formoterol from about 0.1 nM to about 10 µM.

In some embodiments, salmeterol is used in the compositions and methods described herein. Salmeterol **1** exhibits partial agonist activity, which is believed to reduce receptor desensitization and may limit arrestin signaling leading to less receptor down regulation. In some embodiments salmeterol **1** is present as a physiologically acceptable salt and/or solvate thereof. Suitable physiologically acceptable salts of salmeterol **1** include, but are not limited to acid addition salts derived from inorganic and organic acids, such as the hydrochloride, hydrobromide, sulfate, phosphate, maleate, tartrate, citrate, benzoate, 4-methoxybenzoate, 2-hydroxybenzoate, 4-hydroxybenzoate, 4-chlorobenzoate, p-toluenesulphonate, methanesulphonate, ascorbate, salicylate, acetate, fumarate, succinate, lactate, glutarate, gluconate, tricarballylate, hydroxynaphthalenecarboxylate, 1-hydroxy-2-naphthalenecarboxylate, 3-hydroxy-2-naphthalenecarboxylate, oleate, combinations thereof, and the like. In some embodiments salmeterol **1** is provided as the 1-hydroxy-2-naphthalene carboxylate salt (hydroxynaphthoate).

In some embodiments, a suitable tissue concentration of salmeterol **1** for adipose tissue treatment ranges from about 1 pM to about 100 µM, preferably from about 1.0 nM to about 1 µM e.g., about 10 nM to about 1 µM, about 40 nM to about 3 µM, about 0.1 µM to about 1µM, or any other tissue concentration of salmeterol from about 1.0 nM to about 10 uM.

In some embodiments, a long acting selective beta-2 agonist to be administered is formoterol and a therapeutically effective amount of formoterol is about 0.001 to about 100 µg/day, e.g., about 0.001 to about 50, 0.01 to about 1.0, about 0.1 to about 10 , about I to about 20, about 5 to about 40, about 25 to about 75, about 50 to about 100 µg/day of formoterol, or any other dose of formoterol from about 0.001 µg/day to about 100 µg/day.

In some embodiments, a long acting selective beta-2 agonist to be administered is salmeterol and a therapeutically effective amount of salmeterol to be administered is about 0.01 µg/day to about 1000 µg/day, e.g., about 0.1 µg/day to about 100 µg/day, about 1 µg/day to about 100 µg/day, about 10 µg/day to about 100 µg/day, about 50 µg/day to about 100 µg/day, or any other dose of salmeterol from about 0.01 µg/day to about 1000 µg/day.

A "therapeutically effective amount," as used herein, refer to a sufficient amount of an agent (e.g., a long acting beta 2 agonist) or a compound being administered which will relieve to some extent one or more of the symptoms of the disease or condition being treated. The result can be reduction and/or alleviation of the signs, symptoms, or causes of a disease, or any other desired alteration of a biological system. For example, an "effective amount" for therapeutic uses is the amount of the composition including a compound as disclosed herein required to provide a clinically significant decrease in disease symptoms without undue adverse side effects. An appropriate "effective amount" in any individual case may be determined using techniques, such as a dose escalation study. The term "therapeutically effective amount" includes, for example, a prophylactically effective amount. An "effective amount" of a compound disclosed herein, such as a selective beta-2 agonist used alone or in combination with other compounds (e.g., a compound for reducing beta-2 adrenergic receptor desensitization), is an amount effective to achieve a desired pharmacologic effect or therapeutic improvement without undue adverse side effects. It is understood that "an effect amount" or "a therapeutically effective amount" can vary from subject to subject, due to variation in metabolism of beta-2 agonists and compounds used in combination with beta-2 agonists (e.g., glucocorticosteroids), age, weight, general condition of the subject, the condition being treated, the severity of the condition being treated, and the judgment of the prescribing physician.

Some embodiments comprise optically pure isomers of the beta adrenergic agonist(s), which may improve lipolysis and adipogensis inhibition and reduce potential side effects. In some embodiments, these optically pure isomers allow formulations comprising larger loadings of an active ingredient, for example, by eliminating one or more isomers with no physiological effect, a lesser a physiological effect, a negative effect, and/or an undermined physiological effect. Removing the undesired bounds of a racemic mixture isolates the active isomer, or eutomer, thereby allowing more eutomer to be loaded in a give formulation by removing the inactive components.

Two stereogenic centers in a molecule generally generate two diastereomers, referred to herein as (*R**,*R**) and (*R**,*S**), and their enantiomers. Diastereomers are stereoisomers that are not enantiomers, that is, the mirror image of one diastereomer is not superimposable on another diastereomer. Enantiomers are stereoisomers that are mirror images of each other. A racemate is a 1:1 mixture of enantiomers. The enantiomers of the (*R**,*R**) diastereomers are referred to as the (*R*,*R*) and (*S*,*S*) enantiomers, which are mirror images of each other and therefore share some chemical and physical properties, for example melting points. Similarly, the (*R*,*S*) and (*S*,*R*) isomers are enantiomers of the (*R**,*S**) enantiomer. For example, formoterol **2** is available as a racemate of the (*R*,*R*)- and (*S*,*S*)-isomers in a 1:1 ratio, typically as the dihydrate of the fumarate salt. Some embodiments comprise the (*R*,*R*) enantiomer, (*R*,*R*)-formoterol, which is more active as a long-acting beta-2 agonist. Some embodiments comprise optically pure isomers of other beta-2 agonists, for example, (*R*)-salmeterol.

Additionally, in some embodiments, at least one long-acting selective beta-2 agonists is highly lipophilic, thereby providing a pharmaceutical composition with sustained activity in fat tissue. It is believed that high lipid solubility extends the residence time of the beta-2 agonist in the adipose tissue, thereby eliminating or reducing the need for a sustained release and/or controlled release carrier in some embodiments. In formulations comprising a sustained release carrier, for example, a sustained release polymer, the high lipophilicity of the beta-2 agonist facilitates incorporation into the sustained release carrier, as discussed in greater detail below.

Salmeterol **1** and formoterol **2** have high lipid solubilities, which extends their residence time in the adipose tissue and/or in one or more adipose cells. Some embodiments of the composition comprise a highly lipophilic beta agonist, which reduces or eliminates the need for a sustained or controlled release carrier due to partitioning and sequestration in the adipose tissue thereby prolonging the treatment effect. In some embodiments, beta agonists with an oil-water partition coefficient of at least about 1000 or at least about 10,000 to 1 are used. For example, salmeterol **1** is at least 10,000 times more lipophilic than albuterol **5**, a short acting hydrophilic beta agonist. Additionally, salmeterol 1 and formoterol **2** have anti-inflammatory properties, used in the treatment of GO as discussed below. In some embodiments, they also promote favorable extracellular matrix changes and reduce fluid accumulation, which improves the treatment of GO and orbital fat accumulation.

Sustained beta adrenergic activity is further enhanced by preventing desensitization (tachyphylaxis) that can occur with continuous exposure of adipocytes to adrenergic agonists as discussed above. "Compounds that reduce desensitization of beta-adrenergic receptors" (e.g., reduce desensitization of a target tissue to a beta agonist) include all suitable compounds that reduce tolerance of the target tissue to the beta-adrenergic receptor agonists, including glucocorticosteroids and suitable antihistamines, for example, ketotifen, and thyroid hormones, for example T3 and T4. Glucocorticosteroids are also referred herein as "anti-inflammatory steroids," "glucocorticosteroids," and/or "corticosteroids." Glucocorticosteroids are believed to sensitize orbital fat accumulations by increasing the number of beta-2 receptors, thereby favoring lipolysis or fat reduction over fat storage. Glucocorticosteroids may also decrease the number of alpha-2 receptors. Glucocorticosteroids may also stabilize or reduce receptor down regulation especially when given simultaneously with a beta agonist. Of note, Graves disease and GO occur much more commonly in women than men. Estrogen can induce the expression of alpha-2 adrenergic receptors in subcutaneous adipose tissue in women resulting in a ratio of beta-2 receptor to alpha-2 receptor of less than 1. A ratio of beta-2 receptors to alpha-2 receptors greater than about 1 is believed to cause fat reduction rather than fat accumulation in adipocytes. Some embodiments of the composition comprising one or more glucocorticosteroids are effective in treating regions of fat comprising a reduced number of beta-2 receptors and or an increased number of alpha-2 receptors, which are resistant to beta adrenergic stimulation of lipolysis or inhibition of adipogenesis, for example, subcutaneous adipose tissue, especially women.

Thus, glucocorticosteroids or other compounds for reducing desensitization of beta adrenergic receptors is believed to improve lipolysis, adipogenesis inhibition, and/or regional fat reduction during beta agonist exposure. In some embodiments, treatment of adipocytes with a glucocorticosteroid that increases lipolytic activity maintains and/or increases both lipolytic activity and the number of beta-receptors in the target tissue. Examples of suitable corticosteroids include dexamethasone **6**, fluticasone proprionate **7**, budesonide **8**, prednisolone **9**, methylprednisolone **10**,and their analogs. In some embodiments, the glucocorticosteroid is dexamethasone. In some embodiments, the corticosteroid is methylprednisolone.
**6**(9-fluoro- 11,17-dihydroxy-17-(2-hydroxyacetyl)-10,13,16-trimethyl-6,7,8,11,12,14,15,16-octahydrocyclopenta[a]p henanthren-3-one, CAS Reg. No. 50-02-2) and/or fluticasone proprionate **7**.

As discussed above, a suitable compound for reducing beta receptor desensitization may be ketotifen **11**, which is also useful as an antihistamine. Some embodiments of the composition comprise one compound that reduces desensitization of the adipose tissue to the beta-2 agonist.

In some embodiments a plurality of compounds for reducing beta receptor desensitization are used, i.e., a plurality of glucocorticosteroids. Described herein are at least one glucocorticosteroid and the antihistamine ketotifen or an analog of ketotifen.

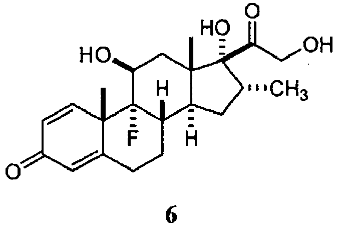

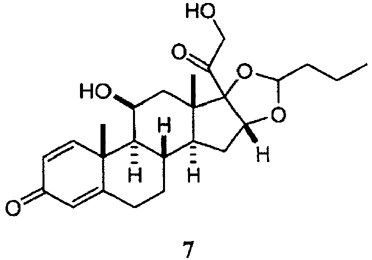

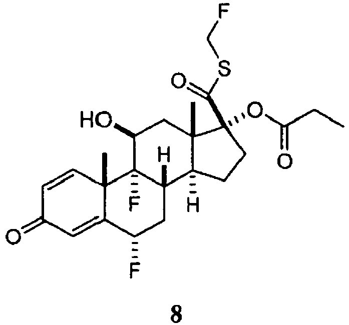

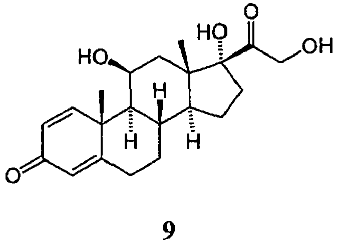

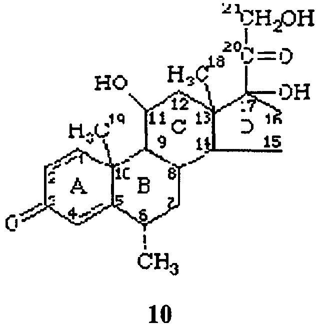

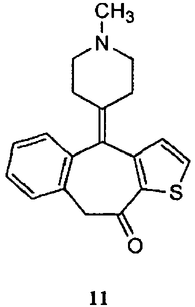

At least one of beta-2 receptor activity or density increases in human orbital adipocytes in response to the anti-inflammatory steroid or ketotifen administration, particularly in the presence of a beta agonist. Increasing beta-2 receptor activity and/or density potentiates the effect of long- and short-acting beta-2 agonists. Thus, the glucocorticosteroid may sensitize orbital fat to the effects of beta-2 receptor stimulation, lipolysis, inhibition of adipogenesis, and/or apoptosis, and/or increases the ratio of beta-2 adrenergic receptors to alpha-2 adrenergic receptors, thereby shifting the balance of the adipose tissue from fat accumulation to fat loss. Beta-2 receptor number is increased or maintained especially with glucocorticoid, ketotifen, or thyroid hormone, especially when co-administered with beta-2 adrenergic agonist.

The addition of an anti-inflammatory such as the glucocorticosteroid or antihistamine has the additional effect of reducing the inflammatory cells and inflammatory response in the orbit. The orbit and orbital fat in GO have a diffuse lymphocytic infiltrate, including lymphoid aggregates or nests. Other tissues of the orbit, such as the extra-ocular muscles, have a similar white blood cell infiltrate. Masts cells may also be present in large numbers. Cytokine secretion by these lymphocytes is consistent with both cellular and humoral medicated responses. Glucocorticosteroids reduce the white cell accumulation, cytokine secretion, and may induce apoptosis of white cells. These effects may be augmented by long acting selective beta-2 agonists. Further, the long acting selective beta-2 agonists may up regulate and stabilize and promote translocation to the nucleus of the receptor for the glucocorticosteroid in white cells, and even the adipocyte, further potentiating the anti-inflammatory effects. Glucocorticosteroid and the long acting beta agonist can stabilize the mast cells and may work additively or synergistically. Ketotifen may also stabilize mast cells and may also inhibit TNF-alpha, a prominent cytokine in lymphocyte medicated cellular inflammatory responses.

Appropriate tissue concentrations of glucocorticosteroids used for the therapeutic methods described herein may range from about 0.001 µM to about 10 mM, e.g., from about 1.0 µM to about 5 mM, from about 40 µM to about 3 mM, from about 100 µM to about 1 mM, or any other tissue concentration of the glucocorticosteroid from about 10 µM to about 10 mM.

In some embodiments, a glucocorticosteroid to be administered is budesonide and the pharmaceutically effective amount of budesonide is about 1.0 to about 320 µg/day, e.g., about 80 to about 300, about 100 to about 280, about 120 to about 260, about 140 to about 240, about 160 to about 220, about 180 to about 200, about 185 to about 195 µg/day of budesonide, or any other dose of budesonide from about 60 to about 320 µg/day.

In some embodiments, the glucocorticosteroid to be administered is fluticasone and the therapeutically effective amount of fluticasone is from about 1.0 to about 500 µg/day, e.g., about 120 to about 480, about 140 to about 460, about 160 to about 440, about 180 to about 420, about 200 to about 400, about 220 to about 380, about 240 to about 360, about 260 to about 340, about 275 to about 310, or about 290 to about 300 µg/day of fluticasone, or any other dose of fluticasone from about 100 to about 500 µg/day.

In some embodiments, the glucocorticosteroid to be administered is methylprednisolone at about 1.0 µg/day to 10,000 µg/day or more, e.g., 50 to 5,000, 100 to 5,000, 500 to 5000, 700 to 3,000, 800 to 2500, 1000 to 2000, or any other dose from about 1.0 to 10,000 µg/day. In some embodiments methylprednisolone succinate may be solubilized in or coadministered with crystalline microparticle methylprednisolone acetate suspension to provide immediate dosing and sustained dosing.

Some embodiments of the composition comprise additional optional ingredients. For example, the orbit of the eye and orbital fat accumulations particularly in the setting of GO contain large amounts of glycosaminoglycans comprise substantially of hyaluronic acid. In some situations, it is advantageous to degrade this hyaluronic acid, for example, to improve the diffusion of the formulation of beta adrenergic agonist and glucocorticosteroid through out the orbit. In addition, degrading the hyaluronic acid may further reduce the orbital tissue mass and reduce orbital edema thereby improving the proptosis and GO condition. Some embodiments of the composition comprise an enzyme such as hyaluronidase, (e.g. recombinant human hyaluronidase, Hylenex, Halozyme Therapeutics, San Diego, CA), which degrades the in the hyaluronic acid.

Some embodiments of the composition comprise one or more anti-lipolytic blocking agents, for example, selective alpha-2 receptor antagonists such as phentolamine **12** (CAS Reg. No. 73-05-2) or yohimbine **13** (CAS Reg. No. 146-48-5) block anti-lipolytic effects in regional fat accumulation. Anti-lipolytic effects in adipocytes and adipose tissue are typically observed in subcutaneous and regional areas of fat accumulation. For example, when exposed to beta agonists, subcutaneous fat has a lower lipolytic rate than visceral fat. Exposing orbital fat to anti-lipolytic blocking agents may improve lipolytic activity in some embodiments.

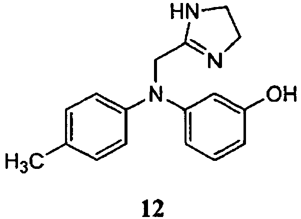

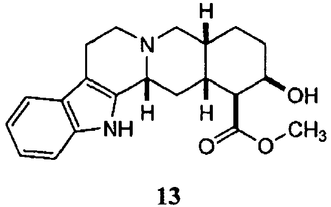

Some embodiments of the composition comprise other adrenergic agents that enhance the effect of the long-acting selective beta-2 agonist. For example, aminophylline **14** (1,3-dimethyl-7*H*-purine-2,6-dione, diethylamine CAS Reg. No. 317-34-0) and theophylline **15** (CAS Reg. No. 58-55-9) are lipolytic agent that block the breakdown of cyclic AMP.

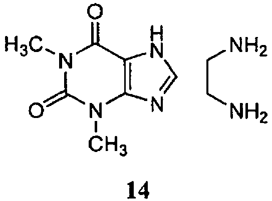

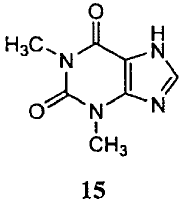

Other optional ingredients increase the secondary signals created by the beta agonist binding. For example, in some embodiments, the composition comprises forskolin **16** (CAS Reg. No. 66575-29-9), which stimulates adenylate cyclase, thereby increasing the synthesis of cyclic AMP initiated by the long-acting beta agonist. The increased concentration of cyclic AMP helps sustain lipolytic activity.

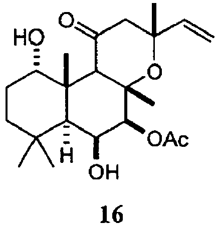

Some embodiments of the composition comprise growth hormone in combination with a long-acting beta agonist and glucocorticosteroid, which appears to stimulate lipolysis.

Others embodiments of the composition further comprises one or more nonselective beta agonists, for example, isoproterenol **4**, and/or short-acting selective beta-2 agonists, for example, terbutaline. Some compositions comprise at least one of an alpha-2 antagonist, or physiologically acceptable salts or solvates thereof.

Embodiments of the composition are formulated for administered by any suitable method, for example, as described in Remington: The Science And Practice Of Pharmacy (21st ed., Lippincott Williams & Wilkins). Exemplary routes of administration include, but are not limited to parenteral, oral, intraocular, intraorbital, periorbital, ophthalmic, retrobulbar, topical, intramuscular, transdermal, sublingual, intranasal, or respiratory. In some embodiments, the composition is formulated for injection of an area at which treatment is desired, for example, in the orbit or orbital fat deposit.

In some embodiments, beta agonists, compounds for preventing beta receptor desensitization, or both are formulated as crystalline microparticle suspensions to prolong release and thereby further sustain adrenergic modulation.

Excipients for injectable formulations can be used. Typically, the excipient is ophthalmically acceptable. In other words, the excipient has substantially no long term or permanent detrimental effect on the eye to which it is administered. Examples of ophthalmically acceptable carriers include water (distilled or deionized water) saline and other aqueous media. The compounds of the invention are preferably soluble in the carrier which is employed for their administration, so that the compounds are administered to the eye in the form of a solution. Alternatively, a suspension of the active compound or compounds (e.g., a suspension of crystalline microparticles) in a suitable carrier may also be employed. In some embodiments, one or more of the beta-2 receptor agonists or glucocorticosteroids are formulated in a liquid carrier, for example, as a solution, a suspension, a gel, and/or an emulsion. Some embodiments comprise any suitable lipophilic, for example, modified oils *(e.g.,* Cremophor® BASF), soybean oil, propylene glycol, polyethylene glycol, derivatized polyethers, combinations thereof, and the like. Some embodiments comprise a microparticulate and/or nanoparticulate carrier for at least one of the beta-2 receptor agonists and/or glucocorticosteroids, as discussed in greater detail below. Some embodiments comprise one or more sustained or controlled release carriers or agents, for example, polymer microspheres. Some embodiments comprise excipients suitable for stable suspensions for micronized particles of the beta-2 receptor agonists or glucocorticosteroids.

Injectable formulations are administered by any means including using a single needle, multiple needles, and/or using a needleless injection device. In some embodiments, a tissue loading dose of the active ingredients formulated in a suitable carrier delivered by injection. In some embodiments, delivery comprises single needle injection. In some embodiments, delivery comprises injection using a multi-needle array, which, in some embodiments, provides a wide dispersion of the formulation in the target tissue. In some embodiments, formulations are injected in a manner that allows dispersal into the appropriate layer of orbital fat. In some embodiments, the formulation is injected small aliquots to reduce the additional volume added to the orbit and may range from about 0.1 mL to about 0.5 mL. In some embodiments the injection device used has a curved needle to facilitate delivery to the retro-orbital or intraconal space.

In some embodiments, the beta-2 agonist and the compound that reduces desensitization are administered, for example injected, as separate formulations, or, alternatively, are administered by separate routes (e.g.,glucocorticosteroid) administered orally followed by injection of a long acting beta-2 agonist). In some embodiments, the compound that reduces desensitization is administered prior to the beta-2 agonist. In other embodiments, the beta-2 agonist is administered prior to the compound that reduces desensitization.

The interval between administration of the compound that reduces desensitization and administration of the beta-2 agonist can be an interval from about 5 minutes to up to 7 days, e.g., 30 minutes, 1 hour, 6 hours, 12 hours, 1 day, 2 day, 3 days, 4 days, 5 days, 6 days, or 7 days, or any other time interval from about 5 minutes to about 7 days. In a preferred embodiment, the compound that reduces desensitization (e.g., a corticosteroid) is administered orally up to about 7 days, e.g, 3 days, 4 days, 5 days, 6 days, 7 days, 8 days, 9 days, or 10 days prior to administering the beta-2 agonist (e.g., by local application of an ophthalmic formulation to the eyes).

In other embodiments, the beta-2 agonist is co-administered (e.g., as part of the same formulation) with the compound that reduces beta receptor desensitization (e.g., a glucocorticosteroid).

In some embodiments a formulation, a subject to be treated is provided a depot formulation, which comprises one or more sustained or controlled release agents for providing a sustained or controlled release of a beta-2 agonist or the compound (e.g., a glucocorticosteroid) for inhibiting desensitization of beta receptors. In such formulations, the beta-2 agonist, the compound for reducing beta receptor desensitization, or both are encapsulated in, bound to, and/or conjugated to the sustained or controlled release agent or carrier. In some embodiments, biocompatible, biodegradable sustained or controlled release formulations provide local tissue activity for weeks to months. Suitable sustained or controlled release agents or carriers include polymers, macromolecules, active ingredient conjugates, hydrogels, contaminations thereof, and the like. Some embodiments of the sustained release carrier target fat, for example, liposomes. Preferably, the sustained release materials are selected to facilitate delivery of a substantially equal amount of the active substance per unit time, particularly over the course of at least about 3 days, more particularly at least about 4 days, to up to one year or greater. Several rounds of injections of the sustained release formulation can be made over time to treat a single area. In some embodiments, sustained release results from formulating the beta-2 agonist or compound for reducing beta receptor desensitization or both as a suspension of crystalline drug microparticles.

In some embodiments, the sustained release agent comprises a polymer, for example, polylactides, polyglycolides, poly(lactide glycolides) polylactic acids, polyglycolic acids, polyanhydrides, polyorthoesters, polyetheresters, polycaprolactones, polyesteramides, polycarbonates, polycyanoacrylates, polyurethanes, polyacrylates, and blends, mixtures, or copolymers of the above, which are used to encapsulate, binds, or conjugate with the active ingredients(s) (*e.g*., beta agonists and/or glucocorticosteroids). Some preferred embodiments of sustained release polymers comprise polyethylene glycol groups to which one or more of the active ingredients is conjugated. In some preferred embodiments, the sustained release agent comprises poly(lactide glycolide) (PLGA, poly(lactic-co-glycolic acid)) copolymer **17**.

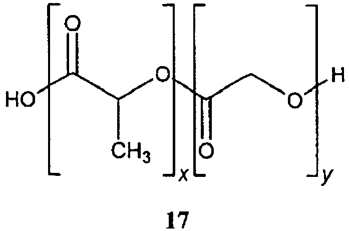

Some embodiments of the sustained release agent comprise one or more hydrogels, including modified alginates. Examples of suitable modified alginates include those disclosed in WO 98/12228. Some embodiments of the sustained release agent comprise an albumin-based nano-particle carrier or excipient.

In some embodiments, a formulation comprising a prepolymer solution is injected into the target tissue site, where it is then polymerized (*e.g*., by photopolymerization) or solidified (*e.g*., by using temperature sensitive gelling materials) *in vivo.*

In some embodiments, the controlled release materials have release characteristics designed for the particular application of tissue reduction. In some embodiments, the sustained release or controlled release agent is formed into microparticles, such as microspheres, which are formulated as an injectable solution and/or gel. In some embodiments, the microparticles range in size from about 10 µm to about 100 µm in diameter and are generally uniform in size. In some embodiments, formulations comprising alginates and/or poly(lactide-co-glycolide)s 17 are provided as an injectable gel or processed into microspheres. In other embodiments the beta-2 agonist or a corticosteroid are formed as crystalline microparticles. Other examples of suitable injectable biodegradable, biocompatible materials suitable for microparticle formation include chitosan, dextran, hydroxyapetite, and silicon.

Microspheres and/or microparticles are formed using any method, including by solvent evaporation and/or emulsion polymerization. In some embodiments, the microspheres have average diameters of from about 5 µm to about 60 µm, preferably, about 20 µm. In some embodiments, PLGA is manufactured with varying ratios of lactide to glycolide depending on the desired rate of release of the active ingredient(s). Because the rate of degradation of this copolymer is proportional to its crystallinity and the proportion of glycolide in the formulation, non-racemic mixtures of the lactide and/or glycolide increase crystallinity and slow the rate of degradation. Higher proportions of glycolide increase the rate of degradation. In some embodiments, a ratio of about 65%-75% lactide to about 25%-35% glycolide provides active ingredients released over from about 2 weeks to about 45 days. In other embodiments, the ratio of lactide to glycolide is from about 0:100 to about 100:0, thereby providing other release rates.

Some embodiments of the microspheres or microparticles comprise hollow and/or porous interiors. In some embodiments, the microspheres comprise a solid or porous outer shell.

In some embodiments, formulations comprising a porous outer shell and/or microsphere exhibit a biphasic release profile of the active ingredient(s) with an initial release burst of the active ingredient(s), followed by a sustained release associated with degradation of the polymeric microspheres. The initial burst loads the tissue with an effective lipolytic/adipogenesis inhibitory and anti-inflammatory concentration of the active ingredient(s), with the subsequent slower release maintaining the desired concentration. In some embodiments, the different microsphere structures and active ingredient release profiles optimize the treatment effect of orbital adipose tissue and adipocytes and through adrenergic receptor modulation and an effect on white blood cell infiltrate to reduce inflammation. In some preferred embodiments, sustained local tissue concentrations of long-acting selective beta-2 adrenergic agents, such as salmeterol **1** and/or formoterol **2** at concentrations of about 1.0 pM to about 10 µM, e.g., about 0. 01 µM to about 10 µM, about 0.1 µM to about 5 µM, 0.5 µM to about 4 µM, or any other concentration from about 0.001 µM to about 10 µM. Sustained local tissue concentrations of glucocorticosteroids may range from about 0.01 uM to 10 mM,.

In some embodiments, one or more of the active ingredients are encapsulated, bound, and/or conjugated to the polymer at a ratio of about 10-12% by mass compared to the polymer microspheres. The amount of active ingredient as a mass percentage of the carrier (*e.g*., microparticles or microspheres) is referred to herein as "active ingredient loading." As used herein, the terms "loaded'' and "loading" refer to active ingredients substantially encapsulated bound, and/or conjugated to a carrier. In some embodiments, the active ingredient loading is up to about 75%. Thus, some preferred formulations comprise one or more beta-2 adrenergically active ingredients, such as salmeterol **1**, formoterol **2**, and/or their physiologically acceptable salts and solvates, loaded on polymer microspheres at about 1 mg to about 20 mg of active ingredient per about 10 to about 200 milligrams of polymer. In some embodiments, a formulation with this active ingredient loading is sufficient for providing from about 15 days to about 45 days of active ingredient release at a concentration suitable to produce lipolysis and/or adipogenesis inhibition. Similarly, glucocorticosteroids budesonide and fluticasone in pharmaceutically acceptable forms may be loaded from about 1 mg to about 20 mg of active ingredient per about 10 to about 200 mg of polymer to produce an anti inflammatory effect.

In some embodiments, two or more active ingredients are loaded into the same microparticle, for example, in a liposome or PLGA. Thus, some embodiments, a polymer encapsulating a glucocorticosteroid in the adrenergic compound is delivered simultaneously to the adipose tissue. Alternatively, the two active ingredients are loaded on separate microparticles. The two types of microspheres are then mixed to obtain a formulation with the desired ratio of beta-receptor agonist and glucocorticosteroid, then administered simultaneously. Alternatively, the two types of microparticles are administered sequentially.

The microspheres comprising the active ingredient(s) are suspended in from about 0.5 mL to 10 mL of an appropriate physiologically acceptable liquid carrier. In some embodiments using separate microspheres of the active ingredients, the microspheres are mixed together in the liquid carrier. In other embodiments, each type of microspheres is separately mixed with a liquid carrier. In some embodiments the liquid carrier may contain a pharmaceutically acceptable form (1.0 to 15 IU/ml) and amount of hyaluronidase. As used herein 1 IU of hyaluronidase produces the same turbidity reduction in a mixture of hyaluronic acid and albumin as 1 I.U. (International Unit) of a standard hyaluronidase preparation. See, e.g., Mathews et al (1966), Methods Enzymol. 8, 654-662. In some embodiments, the microsphere suspension is then injected orbitally or in the retrobulbar space in 0.1 to 0.5 mL aliquots. Alternatively, injections as described above are made separately and sequentially in the same locations using two microsphere formulations encapsulating each active ingredient.

In some embodiments, the glucocorticosteroid, such as dexamethasone **6**, budesonide **8**, and/or fluticasone propionate **7**, also act as anti-inflammatory agents thereby reducing inflammation caused by administration of the formulation, for example, caused by polymers, polymeric microspheres, and/or liposomes in a sustained release formulation.

PLGA **15** microspheres encapsulate hydrophobic compounds more readily than hydrophilic compounds. To increase loading of hydrophilic active ingredients, in some embodiments, the microspheres are modified with polyethylene glycol units, as discussed above. Microspheres of certain sizes are substantially not absorbed into the blood or removal by lymph, thereby providing release of the active ingredient(s) in the desired location. For example, in some embodiments, the microspheres are from about 20 µm to about 200 µm in diameter. In some embodiments, the size of the microsphere also affects the release profile of the active ingredient(s) in the tissue. In general, larger microspheres tend to provide a longer and more uniform release profile.

In an exemplary embodiment, a sustained release formulation comprises about 0.5 milligrams to about 7.5 mg (e.g., about 0.7, 1, 1.5, 2.0, 2.5, 3, 3.5, 4, 4.5, 5, 5.5, 6, 6.5, 7, or any other amount from about 0.5 mg to 7.5 mg) of salmeterol 1 and/or formoterol 2, and about 1.5 mgs to about 7.5 mgs (e.g., about 2, 2.5, 3, 3.5, 4, 4.5, 5, 5.5, 6, 6.5, 7, or any other amount from about 1.5 to about 7.5 mg) of dexamethasone 6, fluticasone propionate 7, and/or budesonide 8 encapsulated in about 100 milligrams of polylactide glycolide (PLGA) 15 copolymer microspheres at a ratio of about 70 lactide:30 glycolide. The amount of each active ingredient in the sustained release formulation depends on the number of days of controlled/sustained release required (e.g., about 3 days, **4** days, 5 days, 6 days, 7 days, 8 days, 9 days, or about 10 days). In some embodiments, the copolymer ratio and active ingredient encapsulation deliver up to about 1.0 µg per day (e.g., about 0.02, 0.04, 0.06, 0.07, 0.1, 0.2, 0.4, 0.5, 0.6, 0.8, or any other amount from about 0.02 µg to about 100.0 µg per day) of salmeterol **1** and/or up to about 0.5 µg (e.g., about 0.02, 0.04, 0.06, 0.07, 0.08, 0.09, 0.1, 0.2, 0.3, 0.4, or any other amount from about about 0.02 µg to about 0.5 µg per day) of formoterol, and up to 5 µg per day (e.g., about 0.2, 0.4, 0.5, 0.7, 0.9, 1.0, 1.5, 2, 2.5, 3, 3.5, 4, 4.5, or any other amount from about 0.2 to about 5 µg per day) of fluticasone and/or budesonide 6 per about 1 mg of copolymer for up to about 30 days. In another exemplary embodiment, the sustained release formulation to be administered comprises methylprednisolone acetate in as a crystalline microparticle suspension with a beta-2 adrenergic agonist. The beta-2 adrenergic agonist may be formulated as solution or may also be formulated as a crystalline mircroparticle suspension. In some embodiments, the sustained release formulation also comprises soluble methylpredinisolone succinate so as to provide immediate effects (due to rapid release) in addition to the sustained effect from the crystalline form of methylprednisolone acetate.

In some embodiments, the one or more beta-2 agonists and methylprednisolone are provided mixed together prior to administration (e.g., injection). In other embodiments, the one or more beta-2 agonists and methylprednisolone are mixed at the time of administration (e.g., injection).

In some embodiments, a selected sustained corticosteroid release formulation (e.g., methylprednisolone crystalline suspension, methylprednisolone solution, or combination of crystalline suspension and solution) is delivered alone up to about 7 days ( e.g., at least 12 hours, 1 day, 2 days, 3 days, 4 days, 5 days, 6 days, or any other time span from about 12 hours to about 7 days) prior to the beta-2 agonist to allow for beta receptor upregulation.

In some embodiments, the subject to be treated is provided a non-sustained release formulation. In some embodiments, the non-sustained release formulation, after a single dose, provides activity of one or more long-acting selective beta-2 agonists for a duration from about four hours to about 24 hours, e.g., 6 hours, 8 hours, 10 hours, 12 hours, 16 hours, 18 hours, 21 hours, or any other duration of beta-2 agonist activity from about four hours to about 24 hours.

In other embodiments, the subject to be treated is provided a non-sustained release formulation comprising short-acting selective beta-2 agonists, which have activities that last less than about four hours (e.g, about 3.5 hours, 3 hours, 2.5 hours, 2 hours, 1.5 hours, 1.3 hours, about 1 hour, 0.5 hours, or any other duration from less than about four hours to about 0.5 hour).

In an exemplary embodiment, a non-sustained release injectable formulation comprises from about 100 µg to about 250 µg (e.g., 105, 110, 125, 150, 175, 190, 200, 210, 225, or any other amount from about 100 µg to about 250 µg) of salmeterol xinafoate and from about 500 µg to about 1000 µg (e.g, 600, 650, 700, 730, 740, 800, 825, 875, 900, 930, 950, or any other amount from about 500 µg to about 1000 pg) of fluticasone propionate formulated in a volume of up to about 10 ml (e.g., 0.3, 0.5, 0.7, 1.1, 1.5, 2, 2.5, 3, 3.5, 4, 5, 6, 7, 8 9, or any other volume from about 0.3 to about 10 ml) of an excipient compatible with administration into the orbit. The excipient concentration may be kept below 1% (e.g., 0.05%, 0.2%, 0.3%, 0.4%, 0.5%, 0.6%, 0.8%, or any other concentration from about 0.05% to less than 1%.

In some embodiments, formulations described herein are delivered transocularly using any suitable method, e.g., as topically applied drops or through a reservoir placed under an eyelid.

In other embodiments, where the formulation to be delivered comprises long-acting beta-2 agonists, such as formoterol 2, salmeterol **1**, or bambuterol **3**, and glucocorticosteroids topical application is suitable. In some embodiments, transdermally deliverable sustained release formulations include a biodegradable, biocompatible active ingredient-polymer formulation or liposome formulation, as discussed above.

Described herein is a method for treating thyroid eye disease by local delivery of a formulation comprising one or more immunosuppressant agents (e.g., rapamycin, sirolimus, or everolimus) to inhibit proliferation of fibroblasts and the conversion of pre-adipocytes into adipocytes. In some instances, the immunosuppressant agents are administered in a sustained release formulation (e.g., a depot formulation) comprising the one or more immunosuppressant agents in a crystalline microparticle suspension. In other instances, the sustained release formulation comprises polymeric microparticles (as described herein) loaded with the one or more immunosuppressants. Administration of the sustained release immunosuppressant formulation can be orbital, periorbital, or retrobulbar injection.

Thyroid hormone may be included in one of the foregoing formulations to up-regulate beta receptors, or increase the beta receptor number on the cell surface, and down-regulate alpha receptors, or decrease the alpha receptor number on the cell surface. Both thyroxine (T4) 18 and triiodothyronine (T3) 19 and stereoisomers of these hormones may be used for this purpose. Levothyroxine is a stereoisomer of thyroxine, which may have a longer half life. These thyroid hormones may be combined with beta agonists and administered by orbital, periorbital, or retrobulbar injection. The one or more thyroid hormones may also be combined with beta agonists in a sustained release formulation, such as a polymer or liposome, as described above, to reduce the administration frequency. T3 and T4 may be combined with selective long acting beta 2 agonists in polymeric microspheres of poly lactide or poly lactide/glycolide for injection and sustained release.

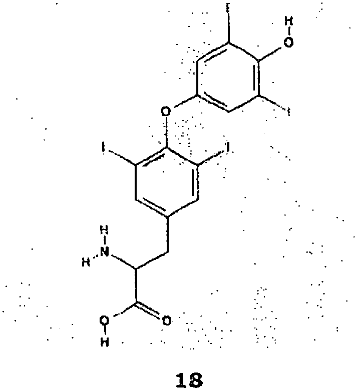

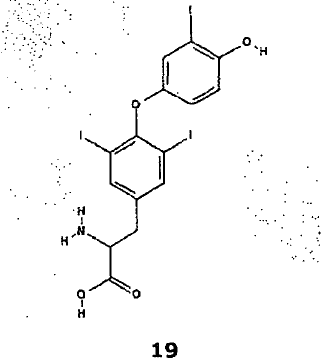

In some embodiments, the above-described formulations further include one or more flavinoids of the flavone and flavinone group (e.g., quercetin and fisetin), which act as inhibitors of cAMP phosphodiesterase and thereby enhance beta-adrenergic signaling.

### EXAMPLES

The following specific examples are to be construed as merely illustrative, and not limitative of the remainder of the disclosure in any way whatsoever. Without further elaboration, it is believed that one skilled in the art can, based on the description herein, utilize the present invention to its fullest extent. Where reference is made to a URL or other such identifier or address, it is understood that such identifiers can change and particular information on the internet can come and go, but equivalent information can be found by searching the internet. Reference thereto evidences the availability and public dissemination of such information.

**Example 1: In vitro Lipolysis Assay of Rat Adivocytes by Beta Agonists and Glucocorticosteroids**

In the *in vitro* lipolysis assay, glycerol was detected in cell culture media via a spectrophotometric measurement after chemical oxidation with hydrogen peroxide. Glycerol was measured over a three hour time period. Levels of lipolysis in cultured human adipocytes were tested after exposure to a beta agonist alone, a glucocorticosteroid alone, or the combination of the two for one or more preincubation periods as described in more detail below.

### Isolation of Pre-adipocytes and Differentiation into Adipocytes:

Human subcutaneous adipocytes were used in the lipolysis assay. Adipose tissue was harvested from liposuction or lipectomy and pre-adipocytes were isolated as follows. Briefly, fat tissue was minced and incubated at 37 °C in Krebs-Ringer bicarbonate buffer containing 1% bovine serum albumin and 0.1% collagenase in an oxygen-rich shaking chamber (5% CO₂; 75 strokes/min) for 1 hour. The suspension was filtered through a 400 µm nylon mesh and centrifuged for 1 min at 100g. The pre-adipocytes in the supernatant were washed twice with and then plated in 96 well plates at a density of cells/well. The pre-adipocytes were cultured in maintenance medium for seven days as they differentiated into adipocytes.

### Reagents

Wash Buffer (Krebs Ringer Buffer (KRB) without serum; [Sigma, K4002-10X1L]) - stored at 4°C
Assay Buffer (KRB with 1% FBS; [FBS from Gibco, 26140-079])- stored at 4°C
Maintenance Medium stored at 4°C
Glycerol Reagent A (Zen-Bio, RGTL-15 or RGTL-40) - after reconstituting, store at 4°C protected from light.
Glycerol stock solution (1 M), prepared by diluting glycerol [Sigma G2025-500ML] in Wash Buffer (no serum) - stored at -20°C.

### Lipolysis Assay:

At -21 hours before the lipolysis assay, medium was removed from each well and replaced with 75 µl of Maintenance Medium containing appropriate drug or DMSO (vehicle) concentrations (see Experimental Design section below). Each test drug or control treatment was applied to 8 wells/group (12 treatment groups per 96-well plate). At -3 hours prior to the lipolysis assay, each well was washed two times with Wash Buffer (200 µl/wash), filled with test or control solutions made up in Assay Buffer (75 µl/well), and then incubated for three hours, i.e., until measurement of glycerol content in the Assay Buffer. For some groups, a drug was only added for the three hour incubation period (see Experimental and Control Groups Below). One hour prior to the assay, seven glycerol standards ranging from 200 uM to 3.125 uM were prepared by serial dilution in Assay Buffer.

The glycerol content of the Assay Buffer from each well following the incubation was used as an index for lipolysis, where an increase in glycerol indicated lipolysis. Glycerol levels were assayed colorimetrically via a commercial glycerol assay kit (Randox Laboratory, United Kingdom) and quantified by comparison to a glycerol serial dilution standard curve (3 µM - 200 µM). Glycerol concentrations for each well were normalized to cell density.

### Experimental Design:

In each of the following control or experimental groups n = 8 corresponding to the glycerol measurements for 8 wells from a 96-well cell culture plate.

| **Table 1** **Summary of *In Vitro* Lipolysis Assay Experimental Design** | | | |
|---|---|---|---|
| **Group(s)** | **18 hour Incubation** | **3 Hour Incubation** | **Results shown in Fig.** |
| **Experiment 1** | | | |
| 1 (negative control) | 0.1% DMSO | 0.1% DMSO | 2⁻ |
| 2-11 | 0.1% DMSO | Formoterol (10⁻¹³ M-10⁻⁴M) | 2 |
| 12 (positive control) | 0.1% DMSO | Isoproterenol 10⁻⁶ M | 2 |

| **Experiment 2** | | | |
|---|---|---|---|
| 13 (negative control) | 0.1% DMSO | 0.1% DMSO | 3^{a} |
| 14-18 | 0.1% DMSO | Salmeterol (10⁻⁸ M- 10⁻⁴ M) | 3 |
| 19 (positive control) | 0.1% DMSO | Isoproterenol 10⁻⁶ M | 3 |

| **Experiment 3** | | | |
|---|---|---|---|
| 20 (negative control) | 0.1% DMSO | 0.1% DMSO | 4^{a} |
| 21-22 | 0.1% DMSO | Budesonide 10⁻¹⁰ M and 10⁻⁷ M | 4 |
| 23-26 | Budesonide (10⁻¹² M - 10⁻⁶ M) | Budesonide (10⁻¹² M - 10⁻⁶ M) | 4 |
| 27 (positive control) | 0.1% DMSO | Isoproterenol 10⁻⁶ M | 4 |

| **Experiment 4** | | | |
|---|---|---|---|
| 28 (negative control) | 0.1% DMSO | 0.1% DMSO | 5^{a} |
| 29 | Salmeterol 10⁻⁶ M | Salmeterol 10⁻⁶ M | 5 |
| 30 | Salmeterol 10⁻⁶ M + Budesonide 10⁻⁶ M | Salmeterol 10⁻⁶ M + Budesonide 10⁻⁶ M | 5 |
| 31 | Salmeterol 10⁻⁸ M | Salmeterol 10⁻⁸ M | 5 |
| 32 | Salmeterol 10⁻⁸ M + Budesonide 10⁻⁶ M | Salmeterol 10⁻⁸ M + Budesonide 10⁻⁶ M | 5 |
| 33 (positive control) | Isoproterenol 10⁻⁶ M | Isoproterenol 10⁻⁶ M | 5 |
| All groups are plotted as fold or % difference relative to negative control group data. | | | |

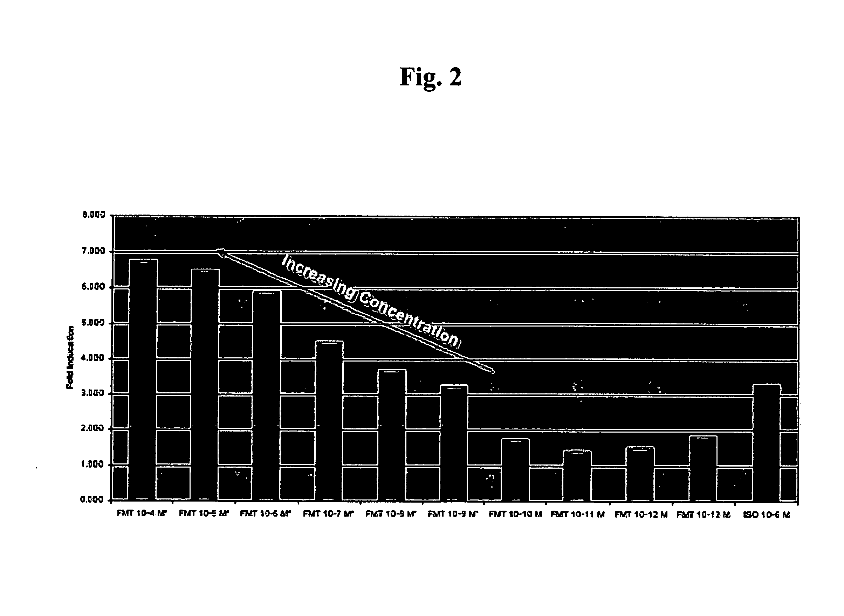

As shown in Fig. 2, the long acting beta-2 adrenergic receptor agonist formoterol induced a dose-dependent increase in lipolysis of greater than six fold after a three hour incubation, which, for concentrations of 10⁻⁶ M and above, was greater than that observed for isoproterenol. Likewise, the long acting beta-2 adrenergic receptor agonist salmeterol also induced a dose-dependent increase in lipolysis after a three hour incubation, although the effect (slightly greater than two fold increase in lipolysis) was not as strong as that observed for Formoterol. Salmeterol-induced lipolysis was equal to or less than that observed for Isoproterenol.

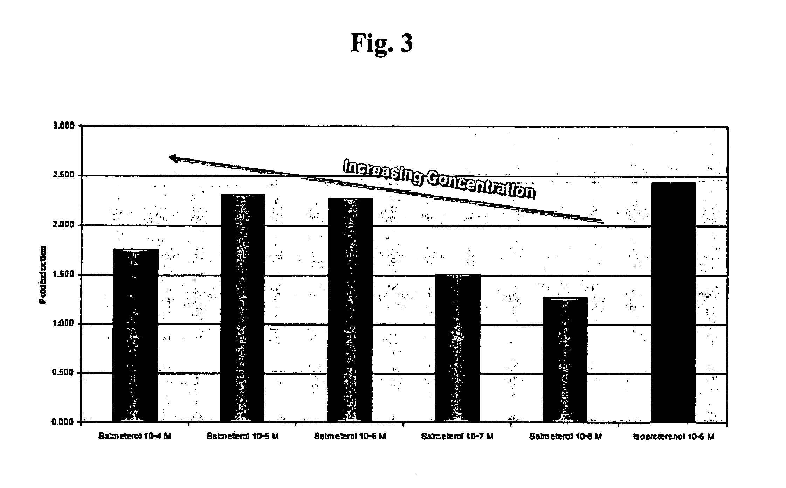

As shown in Fig. 3, the glucocorticosteroid budesonide induced a slight increase (up to about 1.5 fold) in lipolysis after three hours, which was lower than that observed for Isoproterenol ( about 2.5 fold). In contrast, incubation with Budesonide alone for 18 hours actually caused a slight suppression of lipolysis *in vitro.*

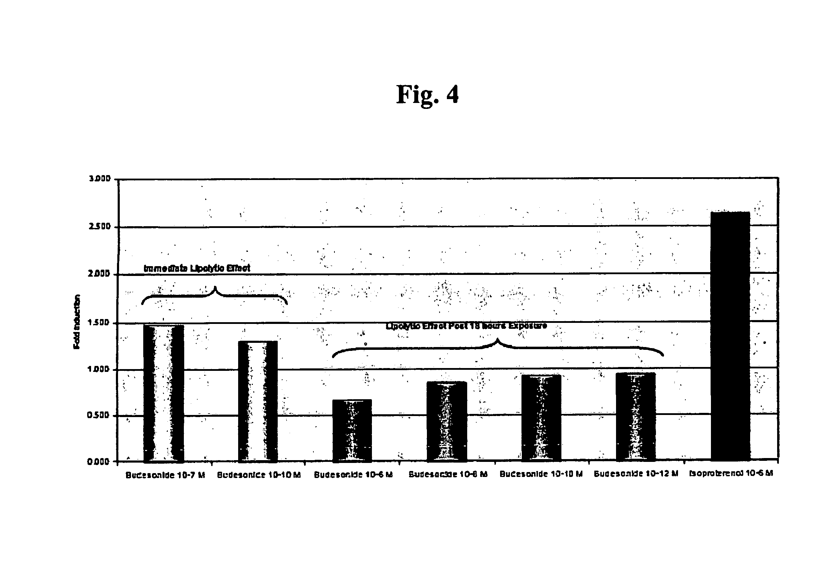

Incubation of adipocytes with Salmeterol (10⁻⁶M) for 18 hours decreased lipolysis (Fig. 4). Similarly, treatment with Isoproterenol for 18 hours resulted in decreased lipolysis. However, when Salmeterol was combined with Budesonide for an 18 hour incubation period, an increase in lipolysis was observed (Fig. 4).

Based on these data, we concluded that formoterol and salmeterol effectively induce lipolysis in cultured adipocytes over a period of three hours. However, Salmeterol actually decreases lipolysis when used for 18 hours, likely due to receptor desensitization or downregulation. Further, in the presence of the glucocorticosteroid Budesonide, Salmeterol is able to induce lipolysis even after 18 hours. Thus, Budesonide can be used to maintain or restore the ability of a beta-2 adrenergic agonist to induce lipolysis over long periods of time, likely by preventing down-regulation of beta-2 adrenergic receptors.

**Example 2: Adipogenesis Inhibition by Beta Agonists and Glucocorticosteroids**

A non-limiting example of such an inhibition of adipogenesis is as follows:

### Cell Culture:

3T3-L 1 preadipocyte cell line (ATCC, Manassas, VA) are plated at 4 X 10⁵ cells per T75 ml flask in Dulbecco's Modified Eagle's Medium (DMEM) with 10% normal calf serum and 1% penicillin/streptomycin antibiotics. Cells are incubated at 37°C, 5% CO₂. After three days, cells are detached by trypsin, counted and resuspended into 24 well plates with 6 X 10⁵ cells per well in 2 mL medium. After 1-2 days, cells are near-confluence and ready for adipogenesis.

### Adipogenesis Materials:

Adipogenesis Initiation Medium: DMEM/10% Fetal Bovine Serum/0.5 mM IBMX/ 1 µM Dexamethasone
Adipogenesis Progression Medium: DMEM/10% Fetal Bovine Serum/10 µg/mL Insulin
Adipogenesis Maintenance Medium: DMEM/10% Fetal Bovine Serum
Negative Control Medium: DMEM/10% Normal Calf Serum

### Adipogenesis Protocol:

1.8 mL of medium is removed from the wells and 2 mL Adipogenesis Initiation Medium added per well. Plates are incubated 48 hours at 37 °C, 5% CO₂. 2 mL of medium is removed and 2 mL of Adipogenesis Progression Medium added per well. Plates are incubated 48 hours at 37 °C, 5% CO₂. 2 mL of medium is removed and 2 mL of Adipogenesis Maintenance Medium added per well. Plates are incubated for at least 48 hours at 37 °C, 5% CO₂. Intracellular lipid droplets accumulate in the cells for at least 5 days.

### Experimental Design:

Prior to adipogenesis, 3T3-L1 preadipocyte cells are pretreated at different stages with a beta 2 agonist and/or glucocorticosteroid. 24 h before addition of Adipogenesis Initiation Medium, cells are treated with the following:
Group 1: No Treatment
Group 2: 10⁻¹⁰ M Salmeterol
Group 3: 10⁻⁸ M Salmeterol
Group 4: 10⁻⁶ M Salmeterol
Group 5: 10⁻⁴ M Salmeterol
Group 6: 10⁻¹⁰ M Salmeterol + 10⁻⁶ M Budesonide
Group 7: 10⁻⁸ M Salmeterol + 10⁻⁶ M Budesonide
Group 8: 10⁻⁶ M Salmeterol + 10⁻⁶ M Budesonide
Group 9: 10⁻⁴ M Salmeterol + 10⁻⁶ M Budesonide
Group 10: 10⁻⁶ M Budesonide
Group 11: 10⁻¹⁰ M Budesonide
Group 12: 10⁻⁶ M Capsaicin, a known adipogenesis inhibitor

Another set of cells is treated with the above group 24 hours prior to addition of Adipogenesis Progression Medium and yet another set is treated 24 hours prior to Adipogenesis Maintenance Medium. In the control set, the cells are treated with with the above group 24 hours prior addition of Negative Control Medium. Two other sets of 12 groups substitute salmeterol for the long acting beta 2 agonist, formoterol, in one set and a short acting beta 2 agonist, albuterol, in the other set.

### Visualization of Intracellular Lipids:

Cells are harvested 5 days after addition of Adipogenesis Maintenance Medium. Cell medium is removed and plates are washed twice with phosphate buffered saline (PBS). 0.5 mL of Oil Red O solution (0.36% Oil Red O in 60% isopropanol) is added per well and plates are incubated at 15 minutes at room temperature. Staining solution is removed and wells are washed three times with 60% isopropanol. Stained plates are then photographed and/or scanned for visual analysis. Lipids are stained red.

### Lipid Quantification:

0.25 mL Dye Extraction Solution (CHEMICON International) is added to the stained wells. Plates are set on an orbital shaker or rocker for 15-30 min. The solution with the extracted dye is transferred to a cuvette and the absorbance read by a spectrophotometer at 520 nm.

### [00117] Example 3: Beta-2 Agonists in Combination with Glucocorticosteroids Decrease Epididymal Fat Pad Mass

We sought to determine if a glucocorticosteroid could reduce fat *in vivo* in a manner consistent with our in *vitro* lipolysis data as described in Example 1. To this end, we measured epididymal fat pad mass in rats treated with the long acting beta-2 adrenergic agonist Formoterol alone and in combination with budesonide.

Male Sprague Dawley rats (~500 g) were anesthetized under 4% isoflurane using a Matrx 3000 vaporizer. The animals, as listed in Table 2 below, were then injected 5 mm anterior to the posterior end of the fat pad with 0.4 ml of vehicle (2% PEG); Formoterol (3.48 µg/ml; dose = 1.39 µg) in the vehicle; or Formoterol (3.48 µg/ml) plus Budesonide (10 µg/ml; dose = 1.39 µg Formoterol and 4 µg Budesonide) in the vehicle. Each animal received a drug treatment on one side and vehicle (2% PEG) treatment on the contralateral side; each group was right-left counterbalanced with respect to drug and vehicle (see Table 2).

| **Table 2: Experimental Design *of In Vivo* Lipolysis Assay** | | | |
|---|---|---|---|
| **Group** | **Animal ID** | **Right epididymal fat pad** | **Left epididymal fat pad** |
| **1** | **1** | Formoterol alone | Vehicle |
| | **2** | Formoterol alone | Vehicle |
| | **3** | Vehicle | Formoterol alone |
| | **4** | Vehicle | Formoterol alone |
| **2** | **5** | Formoterol + Budesonide | Vehicle |
| | **6** | Formoterol + Budesonide | Vehicle |
| | **7** | Vehicle | Formoterol + Budesonide |
| | **8** | Vehicle | Formoterol + Budesonide |

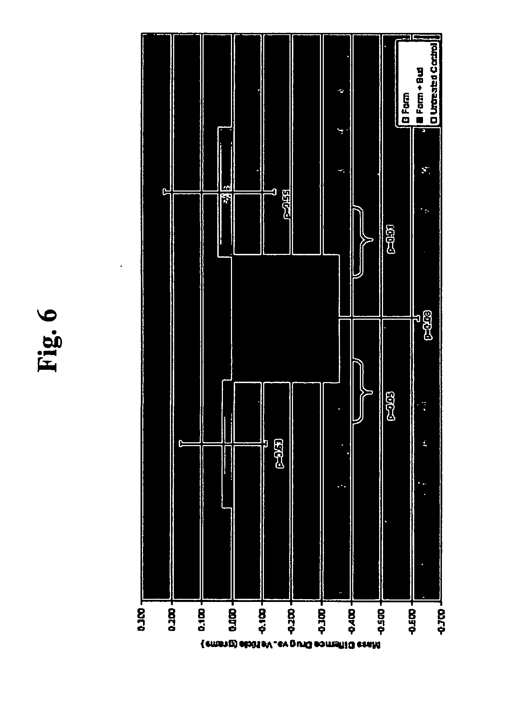

The injections were repeated at 24 and 48 hrs later, for a total of three injections. Twenty four hours after the final injection, animals were euthanized by an i.p.-injected overdose of pentobarbital (150 mg/kg), and the left and right epididymal fat pads from each animal were harvested and weighed (results shown in Table 3 and Fig. 6). Paired t-test and standard t-test was used for statistical analysis.

| **Table 3: Fat Pad Weight Δ (drug-vehicle)** | | |
|---|---|---|
| | **Group** | |
| | **1** | **2** |
| **Animal 1 & 5** | -0.036 | -0.599 |
| **Animal 2 & 6** | -0.138 | -0.115 |
| **Animal 3 & 7** | 0.118 | -0.574 |
| **Animal 4 & 8** | 0.166 | -0.124 |
| | | |
| **Mean** | 0.028 | -0.353 |
| **SD** | 0.140 | 0.270 |

The Formoterol alone and Formoterol + budesonide treatment data showed in Table 3 were analyzed with a paired Student t-test, the results of which are shown in Table 4.

| **Table 4: Statistical Analysis of Fat Pad Weight A (drug-vehicle) following Formoterol alone or Formoterol + Budesonide Treatment** | |
|---|---|
| **Groups** | **Two-tailed p value** |
| Group 1 (Formoterol) vs vehicle | 0.63 |
| Group 2 (Formoterol + Budesonide) vs vehicle | 0.0792 |

As shown in Table 4, Group 1 animals (treated with Formoterol alone) showed differences and variability consistent with naïve untreated control. The mean treatment effect for Formoterol alone was + 0.028 g ± 0.140 g. Statistical analysis yielded a p value of 0.63 consistent with no trend toward a treatment effect. On the other hand, Group 2 animals (treated with Formoterol + Budesonide) showed a treatment effect. The mean treatment effect was -0.353 ± 0.270 with a p value = 0.079.

We also performed a statistical analysis (Student t-test) for significant differences between the effect of the single and combination drug treatments, and also for a significant difference between the combination treatment versus untreated control animals. The results of these statistical analyses are shown in Table 5.

| **Table 5: Statistical Analysis of Differences in Effects Formoterol Alone versus Formoterol + Budesonide; and Formoterol + Budesonide versus Untreated** | |
|---|---|
| **Comparison (Mean difference in Epi fat pad weight)** | **Two-tailed P value** |
| Group 2 (Formoterol + Budesonide) vs Group 1 (Formoterol alone) | 0.05 |
| Group 2 vs. untreated | 0.0131 |

As shown in Table 5 and Fig. 6, there was statistical difference between Group 2 and Group I (p = 0.05). There was a significant difference in the mean fat pad mass reduction in Group 2 versus untreated animals with a p=0.013.

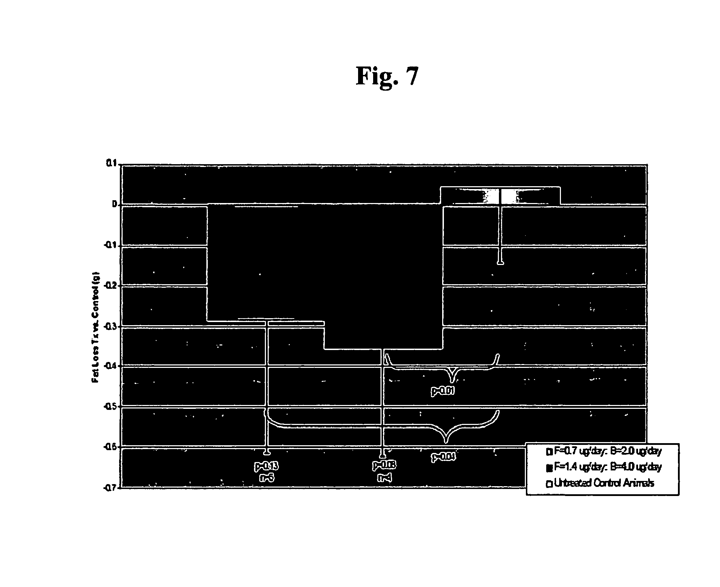

In a follow-up experiment, we examined the ability of formoterol plus budesonide to reduce fat pad mass in the above-described assay. As shown in Fig. 7, administering a dose combination of 1.4 µg/day formoterol + 4.0 µg/day budesonide resulted in a significant difference in mean epididymal mass difference in the treated versus untreated group (p = 0.01). Likewise, even at a lower dose combination (0.7 µg/day formoterol + 2.0 µg/day budesonide, achieved by every other day dosing), the difference between the combination-treated and untreated control animals was significant (p = 0.04).

Based on these data and their analysis, we concluded that a combination of a long acting beta-2 agonist (e.g., Formoterol) and a glucocorticosteroid (e.g., Budesonide) are likely to be effective for inducing lipolysis and fat reduction *in vivo.*

**Example 4: Clinical Testing for Treatment of Graves' Ophthalmopathy with Compositions Comprising Beta Agonist and Glucocorticosteroid**

A non-limiting example of such a clinical testing for treatment of Graves' Ophthalmopathy is as follows:

### Patient Selection:

Patients are to be 18 years of age or above and have no hypersensitivity to the administered drugs. They are diagnosed with proptosis symptoms associated with Graves' Ophthalmopathy by ultrasonography, exophthalmometry, MRI, or computerized tomography. In particular, patients are chosen with unilateral or bilateral proptosis of 0.5 mm or 3mm or more, with or without other symptoms. Patients may also exhibit diplopia, limitation of eye movement in extreme positions, and evident restriction of movement, corneal ulcerations, pain, cosmetic deformity, and low quality of life. Patients may have undergone thyroidectomy for hyperthyroidism. Other steroid therapies should be not used for treatment of hyperthyroidism. All studies are to be performed with institutional ethics committee approval and patient consent.

### Study Design:

Test 1: This is a multicenter, dose escalation study of the combination therapy of salmeterol, a long acting beta 2 agonist with the budesonide, a glucocorticosteroid. Patients receive an injection administration of a parenteral composition of the drug daily. Patients who do not achieve proptosis improvement or partial or complete response but who have stable disease after 1 week of therapy will receive an additional 1 week of therapy at a higher dose than what is originally assigned. Cohorts of 3-6 patients receive escalating doses of the combination drug until the maximum tolerated dose (MTD) is determined. The MTD is defined as the dose preceding that at which 2 of 3 or 2 of 6 patients experience dose-limiting toxicity.

Test 2: This is a randomized, multicenter study. The study length is 60 days. Patients are randomized to 1 of 18 treatment groups. For group 1, patients are given salmeterol of formoterol alone daily at MTD. For group 2, patients are given budesonide alone daily at MTD. For group 3, patients are given salmeterol and budesonide concurrently at MTD. For Group 4, patients are given salmeterol or formoterol daily, and budesonide on every other day. For Group 5, patients are given budesonide daily, and salmeterol or formoterol on every other day. For Group 6, patients are given budesonide on every odd day and salmeterol on every even day. Groups 7-12 have the same dosing regime as 1-6 except the dosage is at one-fourth MTD. Groups 13-18 also have the same dosing regime as 1-6 except the dosage is at one-tenth MTD. In addition to the treatment groups, a control group is left untreated.

### Endpoint Assessment:

Patients are assessed for reduction of proptosis and decrease of orbital fat volume and extraocular muscles at the conclusion of the study. Improvement in eyelid closure and ocular movement is also assessed. A 20% reduction within 60 days is presumed as a positive outcome.

### Example 5: Pharmaceutical Compositions

Non-limiting examples of such pharmaceutical compositions are as follows:

### Parenteral Compositions

Example 5A. To prepare a parenteral pharmaceutical composition suitable for administration by injection, about 100 µg of a water-soluble salt of a formoterol and about 3 mg of ketotifen is dissolved in DMSO and then mixed with 10 mL of 0.9% sterile saline. The mixture is incorporated into a dosage unit form suitable for administration by injection.

Example 5B. To prepare a parenteral pharmaceutical composition suitable for administration by injection, about 50 µg of a water-soluble salt of a salmeterol and about 100 µg of fluticasone proprionate is dissolved in DMSO and then mixed with 10 mL of 0.9% sterile saline containing about 20% v/v PEG-400. Hyaluronidase is added to the mixture to a final concentration of 8 IU/ml. The resulting mixture is incorporated into a dosage unit form suitable for administration by injection.

Example 5C. To prepare a parenteral pharmaceutical composition suitable for administration by injection, about 50 to 100 µg of a water-soluble salt of a salmeterol is dissolved in DMSO and then mixed with 10 mL of 0.9% sterile saline containing about 20% v/v PEG-400. The mixture is incorporated into a dosage unit form suitable for administration by injection.

Example 5D. To prepare a parenteral pharmaceutical composition suitable for administration by injection, about 50 µg of a water-soluble salt of fluticasone proprionate is dissolved in DMSO and then mixed with 10 mL of 0.9% sterile saline. Hyaluronidase is added to the mixture to a final concentration of 10 IU/ml. The resulting mixture is incorporated into a dosage unit form suitable for administration by injection.

### Tropical Gel Compositions

Example 5E. To prepare a pharmaceutical topical gel composition, about 100 mg of salmeterol and about 100 mg of prednisolone is mixed with 1.75 g of hydroxypropyl celluose, 10 mL of propylene glycol, 10 mL of isopropyl myristate and 100 mL of purified alcohol USP. The resulting gel mixture is then incorporated into containers, such as tubes, which are suitable for topical administration.

Example 5F. To prepare a pharmaceutical topical gel composition, about 100 mg of formoterol and about 100 mg of budesonide is mixed with about 10 mg of hyaluronidase, 1.75 g of hydroxypropyl celluose, 10 mL of propylene glycol, 10 mL of isopropyl myristate and 100 mL of purified alcohol USP. The resulting gel mixture is then incorporated into containers, such as tubes, which are suitable for topical administration.

Example 5G. To prepare a pharmaceutical topical gel composition, about 100 mg of salmeterol is mixed with about 10 ml of PEG-400, 1.75 g of hydroxypropyl celluose, 10 mL of isopropyl myristate and 100 mL of purified alcohol USP. The resulting gel mixture is then incorporated into containers, such as tubes, which are suitable for topical administration.

Example 5H. To prepare a pharmaceutical topical gel composition, about 100 mg of prednisolone is mixed with about 10 ml of PEG-400, 1.75 g of hydroxypropyl celluose, 10 mL of isopropyl myristate and 100 mL of purified alcohol USP. The resulting gel mixture is then incorporated into containers, such as tubes, which are suitable for topical administration.

### Ophthalmic Solution Compositions

Example 51. To prepare a pharmaceutical ophthalmic solution composition, about 100 mg of a compound of salmeterol and about 100 mg of budesonide is mixed with 0.9 g of NaCl in 100 mL of purified water and filtered using a 0.2 micron filter. The resulting isotonic solution is then incorporated into ophthalmic delivery units, such as eye drop containers, which are suitable for ophthalmic administration.

Example 5J. To prepare a pharmaceutical ophthalmic solution composition, about 100 mg of a compound of formoterol and about 100 mg of budesonide is mixed with 0.9 g of NaCl in 100 mL of about 10% v/v PEG-400 in purified water and filtered using a 0.2 micron filter. The resulting isotonic solution is then incorporated into ophthalmic delivery units, such as eye drop containers, which are suitable for ophthalmic administration.

Example 5K. To prepare a pharmaceutical ophthalmic solution composition, about 100 mg of a compound of formoterol and about hyaluronidase (to a final concentration of 10 IU/ml) is mixed with 0.9 g of NaCl in 100 mL of about 10% v/v PEG-400 in purified water and filtered using a 0.2 micron filter. The resulting isotonic solution is then incorporated into ophthalmic delivery units, such as eye drop containers, which are suitable for ophthalmic administration.

Example 5L. To prepare a pharmaceutical ophthalmic solution composition, about 100 mg of a compound of ketotifen is mixed with 0.9 g of NaCl in 100 mL of about 20% v/v PEG-400 in purified water and filtered using a 0.2 micron filter. The resulting isotonic solution is then incorporated into ophthalmic delivery units, such as eye drop containers, which are suitable for ophthalmic administration.

### Oral Compositions

Example 5M To prepare a pharmaceutical oral composition, about 100 mg of a compound of prednisolone is mixed with 750 mg of starch. The mixture is incorporated into an oral dosage unit for, such as a hard gelatin capsule, which is suitable for oral administration.

Example 5N To prepare a pharmaceutical oral composition, about 50 mg of a compound of budesonide is mixed with 375 mg of gelatin. The mixture is incorporated into an oral dosage unit for, such as a hard gelatin capsule, which is suitable for oral administration.

Example 50 To prepare a pharmaceutical oral composition, about 200 mg of a compound of ketotifen is mixed with 1500 mg of hydroxypropylmethylcellulose. The mixture is incorporated into an oral dosage unit for, such as a hard gelatin capsule, which is suitable for oral administration.

Example 5P To prepare a pharmaceutical oral composition, about 50 mg of a compound of fluticasone proprionate is mixed with 600 mg of starch. The mixture is incorporated into an oral dosage unit for, such as a hard gelatin capsule, which is suitable for oral administration.

**Example 6: Beta Agonists and Glucocorticosteroid Administration Regimens** Non-limiting examples of such administration regimens are as follows:
Example 6A. A patient suffering from Graves' Ophthalmopathy is administered a therapeutically effective amount of composition 5C on day I and every subsequently odd-numbered day of treatment. On even-numbered days, the patient is administered a therapeutically effective amount of composition 5D.
Example 6B. A patient suffering from Graves' Ophthalmopathy is administered a therapeutically effective amount of composition 5D on day 1 and every subsequently odd-numbered day of treatment. On even-numbered days, the patient is administered a therapeutically effective amount of composition 5C.
Example 6C. A patient suffering from Graves' Ophthalmopathy is administered a therapeutically effective amount of composition 5C daily. On even-numbered days, the patient is administered a therapeutically effective amount of composition 5D.
Example 60. A patient suffering from Graves' Ophthalmopathy is administered a therapeutically effective amount of composition 5D daily. On even-numbered days, the patient is administered a therapeutically effective amount of composition 5C.
Example 6E. A patient suffering from Graves' Ophthalmopathy is administered a therapeutically effective amount of composition 5C on day 1 followed by a holiday of two days. On the second holiday, the patient is administered a therapeutically effective amount of composition 5D. This administration is then repeated.
Example 6F. A patient suffering from Graves' Ophthalmopathy is administered a therapeutically effective amount of composition 5D on day 1 followed by a holiday of two days. On the second holiday, the patient is administered a therapeutically effective amount of composition 5C. This administration is then repeated.
Example 6G. A patient suffering from Graves' Ophthalmopathy is administered a therapeutically effective amount of composition 5M on day 1 and every subsequently odd-numbered day of treatment. On even-numbered days, the patient is administered a therapeutically effective amount of composition 5C.
Example 6H. A patient suffering from Graves' Ophthalmopathy is administered a therapeutically effective amount of composition 5C daily. On even-numbered days, the patient is administered a therapeutically effective amount of composition 5M.

## Claims

1. A therapeutically effective amount of at least one beta adrenergic receptor agonist; and
a therapeutically effective amount of at least one g lucocorticosteroid;
for use in the reduction of orbital fat accumulation in a patient in need thereof.

2. The therapeutically effective amounts of the agents for the use of claim 1, wherein the patient has proptosis.

3. The therapeutically effective amounts of the agents for the use of claim 2, wherein the proptosis is bilateral proptosis.

4. The therapeutically effective amounts of the agents for the use of any of claims 1-3, wherein the patient suffers from thyroid eye disease.

5. The therapeutically effective amounts of the agents for the use of any of claims 1-4, wherein the at least one glucocorticosteroid is administered before the at least one beta adrenergic agonist.

6. The therapeutically effective amounts of the agents for the use of any of claims 1-5, wherein the at least one glucocorticosteroid is administered about 3 days to about 7 days before the at least one beta adrenergic agonist.

7. The therapeutically effective amounts of the agents for the use of any of claims 1-6, wherein the at least one glucocorticosteroid is administered by an intraocular, intraorbital, ophthalmic, periorbital, retrobulbar, or intraconal route of administration.

8. The therapeutically effective amounts of the agents for the use of any of claims 1-7, wherein the at least one beta adrenergic agonist comprises a long-acting beta adrenergic agonist.

9. The therapeutically effective amounts of the agents for the use of any of claims 1-8, wherein the at least one beta adrenergic agonist comprises a beta adrenergic agonist that is selective for the beta-2 adrenergic receptor.

10. The therapeutically effective amounts of the agents for the use of any of claims 1-9, wherein the at least one beta adrenergic agonist comprises salmeterol, formoterol, or any salt or combination thereof.

11. The therapeutically effective amounts of the agents for the use of any of claims 1-10, wherein the at least one beta adrenergic agonist comprises salmeterol and the therapeutically effective amount of salmeterol is about 0.01 µg/day to about 100 µg/day, optionally wherein the at least one beta adrenergic agonist comprises formoterol and the therapeutically effective amount of formoterol is about 0.001 µg/day to about 50 µg/day.

12. The therapeutically effective amounts of the agents for the use of any of claims 1-11, wherein the at least one glucocorticosteroid comprises dexamethasone, prednisolone, methylprednisolone, fluticasone, budesonide, or any combination thereof.

13. The therapeutically effective amounts of the agents for the use of any of claims 1-12, further comprising an immunosuppressant agent wherein the immunosuppressant agent is administered by an intraocular, intraorbital, ophthalmic, periorbital, retrobulbar, or intraconal route prior to the administration of at least one compound, optionally wherein the immunosuppressant agent is present in a therapeutically effective amount which is administered in the form of crystalline microparticle suspension.

14. The therapeutically effective amounts of the agents for the use of any of claims 1-13, wherein the use sensitizes orbital fat accumulation.

15. A preparation comprising the therapeutically effective amounts of the agents for the use of any of claims 1-14, wherein the composition comprises a mixture of at least one long-acting beta adrenergic agonist and at least one short-acting beta adrenergic agonist; and optionally the composition further comprises a therapeutically effective amount of hyaluronidase.

16. A preparation comprising the therapeutically effective amounts of the agents for the use of any of claims 1-14, wherein the at least one beta adrenergic agonist comprises salmeterol, formoterol, bambuterol, eformoterol, isoproterenol, albuterol, or fenoterol, optionally wherein either (i) the composition comprises salmeterol and the therapeutically effective amount of salmeterol is about 0.01 ug/day to about 100 µg/day; (ii) the composition comprises formoterol and the therapeutically effective amount of formoterol is about 0.001 µg/day to about 50 µg/day; or (iii) the administration of the composition is parenteral, oral, intraocular, intraorbital, intraconal, ophthalmic, retrobulbar, topical, intramuscular, transdermal, sublingual, intranasal, or respiratory.

17. The therapeutically effective amounts of the agents for the use of any of claims 1-16, wherein the patient suffers from a disease involving orbital fat accumulation.

18. The therapeutically effective amounts of the agents for the use of any of claims 1-17, where the preparation comprises salmeterol xinafoate.

19. The therapeutically effective amounts of the agents for the use of any of claims 1-18, where the beta-adrenergic receptor agonist is formoterol fumarate.

20. The therapeutically effective amounts of the agents for the use of claims 1-19, where the gluescenticesteroid is fluticasone propionate.

21. The therapeutically effective amounts of the agents for the use of claims 1-20, wherein said at least one beta adrenergic receptor agonist, and said at least one glucorticosteroid are formulated for administration to said patient in an amount sufficient to achieve reduction of orbital fat accumulation.

22. The therapeutically effective amounts of the agents for the use of any of claims 1-20, wherein said at least one beta adrenergic receptor agonist and said at least one glucocorticosteroid are formulated as a combined preparation.

## Patentansprüche

1. Eine therapeutisch wirksame Menge von wenigstens einem beta-adrenergen Rezeptoragonisten; und
eine therapeutisch wirksame Menge von wenigstens einem Glucocorticosteroid;
zur Verwendung bei der Verringerung der Ansammlung von orbitalem Fett bei einem Patienten, der dessen bedarf.

2. Die therapeutisch wirksamen Mengen der Mittel für die Verwendung nach Anspruch 1, wobei der Patient Proptose aufweist.

3. Die therapeutisch wirksamen Mengen der Mittel für die Verwendung nach Anspruch 2, wobei die Proptose eine bilaterale Proptose ist.

4. Die therapeutisch wirksamen Mengen der Mittel für die Verwendung nach irgendeinem der Ansprüche 1 - 3, wobei der Patient an einer thyroidalen Augenkrankheit (thyroid eye disease) leidet.

5. Die therapeutisch wirksamen Mengen der Mittel für die Verwendung nach irgendeinem der Ansprüche 1 - 4, wobei das wenigstens eine Glucocorticosteroid vor dem wenigstens einen beta-adrenergen Agonisten verabreicht wird.

6. Die therapeutisch wirksamen Mengen der Mittel für die Verwendung nach irgendeinem der Ansprüche 1 - 5, wobei das wenigstens eine Glucocorticosteroid ca. 3 Tage bis ca. 7 Tage vor dem wenigstens einen beta-adrenergen Agonisten verabreicht wird.

7. Die therapeutisch wirksamen Mengen der Mittel für die Verwendung nach irgendeinem der Ansprüche 1 - 6, wobei das wenigstens eine Glucocorticosteroid über einen intraokularen, intraorbitalen, ophthalmischen, periorbitalen, retrobulbären oder intrakonalen Verabreichungsweg verabreicht wird.

8. Die therapeutisch wirksamen Mengen der Mittel für die Verwendung nach irgendeinem der Ansprüche 1 - 7, wobei der wenigstens eine beta-adrenerge Agonist einen lang wirkenden beta-adrenergen Agonisten umfasst.

9. Die therapeutisch wirksamen Mengen der Mittel für die Verwendung nach irgendeinem der Ansprüche 1 - 8, wobei der wenigstens eine beta-adrenerge Agonist einen beta-adrenergen Agonisten umfasst, der für den beta-2-adrenergen Rezeptor selektiv ist.

10. Die therapeutisch wirksamen Mengen der Mittel für die Verwendung nach irgendeinem der Ansprüche 1 - 9, wobei der wenigstens eine beta-adrenerge Agonist Salmeterol, Formoterol oder irgendein Salz oder eine Kombination von diesen umfasst.

11. Die therapeutisch wirksamen Mengen der Mittel für die Verwendung nach irgendeinem der Ansprüche 1 - 10, wobei der wenigstens eine beta-adrenerge Agonist Salmeterol umfasst und die therapeutisch wirksame Menge an Salmeterol ca. 0,01 µg/Tag bis ca. 100 µg/Tag beträgt, wobei gegebenenfalls der wenigstens eine beta-adrenerge Agonist Formoterol umfasst und die therapeutisch wirksame Menge an Formoterol ca. 0,001 µg/Tag bis ca. 50 µg/Tag beträgt.

12. Die therapeutisch wirksamen Mengen der Mittel für die Verwendung nach irgendeinem der Ansprüche 1 - 11, wobei das wenigstens eine Glucocorticosteroid Dexamethason, Prednisolon, Methylprednisolon, Fluticason, Budesonid oder irgendeine Kombination von diesen umfasst.

13. Die therapeutisch wirksamen Mengen der Mittel für die Verwendung nach irgendeinem der Ansprüche 1 - 12, die weiterhin ein immunsupprimierendes Mittel umfassen, wobei das immunsupprimierende Mittel vor der Verabreichung wenigstens einer Verbindung über einen intraokularen, intraorbitalen, ophthalmischen, periorbitalen, retrobulbären oder intrakonalen Weg verabreicht wird, wobei gegebenenfalls das immunsupprimierende Mittel in einer therapeutisch wirksamen Menge vorliegt, welche in der Form einer kristallinen Mikropartikel-Suspension verabreicht wird.

14. Die therapeutisch wirksamen Mengen der Mittel für die Verwendung nach irgendeinem der Ansprüche 1 - 13, wobei die Verwendung die Ansammlung von orbitalem Fett sensibilisiert.

15. Ein Präparat, welches die therapeutisch wirksamen Mengen der Mittel für die Verwendung nach irgendeinem der Ansprüche 1 - 14 umfasst, wobei die Zusammensetzung eine Mischung von wenigstens einem lang wirkenden beta-adrenergen Agonisten und wenigstens einem kurz wirkenden beta-adrenergen Agonisten umfasst und wobei die Zusammensetzung gegebenenfalls weiterhin eine therapeutisch wirksame Menge an Hyaluronidase umfasst.

16. Ein Präparat, welches die therapeutisch wirksamen Mengen der Mittel für die Verwendung nach irgendeinem der Ansprüche 1 - 14 umfasst, wobei der wenigstens eine beta-adrenerge Agonist Salmeterol, Formoterol, Bambuterol, Eformoterol, Isoproterenol, Albuterol oder Fenoterol umfasst, wobei gegebenenfalls entweder (i) die Zusammensetzung Salmeterol umfasst und die therapeutisch wirksame Menge an Salmeterol ca. 0,01 µg/Tag bis ca. 100 µg/Tag beträgt; (ii) die Zusammensetzung Formoterol umfasst und die therapeutisch wirksame Menge an Formoterol ca. 0,001 µg/Tag bis ca. 50 µg/Tag beträgt; oder (iii) die Verabreichung der Zusammensetzung parenteral, oral, intraokular, intraorbital, introkonal, ophthalmisch, retrobulbär, topisch, intramuskulär, transdermal, sublingual, intranasal oder respiratorisch erfolgt.

17. Die therapeutisch wirksamen Mengen der Mittel für die Verwendung nach irgendeinem der Ansprüche 1 - 16, wobei der Patient an einer Krankheit leidet, welche eine Ansammlung von orbitalem Fett beinhaltet.

18. Die therapeutisch wirksamen Mengen der Mittel für die Verwendung nach irgendeinem der Ansprüche 1 - 17, wobei das Präparat Salmeterolxinafoat umfasst.

19. Die therapeutisch wirksamen Mengen der Mittel für die Verwendung nach irgendeinem der Ansprüche 1 - 18, wobei der beta-adrenerge Rezeptoragonist Formoterolfumarat ist.

20. Die therapeutisch wirksamen Mengen der Mittel für die Verwendung nach irgendeinem der Ansprüche 1 - 19, wobei das Glucocorticosteroid Fluticasonpropionat ist.

21. Die therapeutisch wirksamen Mengen der Mittel für die Verwendung nach irgendeinem der Ansprüche 1 - 20, wobei der wenigstens eine beta-adrenerge Rezeptoragonist und das wenigstens eine Glucocorticosteroid zur Verabreichung an den Patienten in einer Menge formuliert sind, die ausreicht, um eine Verringerung der Ansammlung von orbitalem Fett zu erzielen.

22. Die therapeutisch wirksamen Mengen der Mittel für die Verwendung nach irgendeinem der Ansprüche 1 - 20, wobei der wenigstens eine beta-adrenerge Rezeptoragonist und das wenigstens eine Glucocorticosteroid als ein kombiniertes Präparat formuliert sind.

## Revendications

1. Quantité thérapeutiquement efficace d'au moins un agoniste de récepteur β-adrénergique ; et quantité thérapeutiquement efficace d'au moins un glucocorticostéroïde ; pour l'utilisation dans la réduction de l'accumulation de graisse orbitaire chez un patient en ayant besoin.

2. Quantités thérapeutiquement efficaces des agents pour l'utilisation selon la revendication 1, où le patient a une proptose.

3. Quantités thérapeutiquement efficaces des agents pour l'utilisation selon la revendication 2, où la proptose est une proptose bilatérale.

4. Quantités thérapeutiquement efficaces des agents pour l'utilisation selon l'une quelconque des revendications 1 à 3, où le patient souffre d'une maladie oculaire thyroïdienne.

5. Quantités thérapeutiquement efficaces des agents pour l'utilisation selon l'une quelconque des revendications 1 à 4, où le au moins un glucocorticostéroïde est administré avant le au moins un agoniste β-adrénergique.

6. Quantités thérapeutiquement efficaces des agents pour l'utilisation selon l'une quelconque des revendications 1 à 5, où le au moins un glucocorticostéroïde est administré d'environ 3 jours à environ 7 jours avant le au moins un agoniste β-adrénergique.

7. Quantités thérapeutiquement efficaces des agents pour l'utilisation selon l'une quelconque des revendications 1 à 6, où le au moins un glucocorticostéroïde est administré par une voie d'administration intraoculaire, intra-orbitaire, ophtalmique, périorbitaire, rétrobulbaire, ou intra-conique.

8. Quantités thérapeutiquement efficaces des agents pour l'utilisation selon l'une quelconque des revendications 1 à 7, où le au moins un agoniste β-adrénergique comprend un agoniste β-adrénergique à action prolongée.

9. Quantités thérapeutiquement efficaces des agents pour l'utilisation selon l'une quelconque des revendications 1 à 8, où le au moins un agoniste β-adrénergique comprend un agoniste β-adrénergique qui est sélectif pour le récepteur β2-adrénergique.

10. Quantités thérapeutiquement efficaces des agents pour l'utilisation selon l'une quelconque des revendications 1 à 9, où le au moins un agoniste β-adrénergique comprend le salmétérol, le formotérol, ou n'importe quel sel ou combinaison de ceux-ci.

11. Quantités thérapeutiquement efficaces des agents pour l'utilisation selon l'une quelconque des revendications 1 à 10, où le au moins un agoniste β-adrénergique comprend du salmétérol et la quantité thérapeutiquement efficace de salmétérol est d'environ 0,01 µg/jour à environ 100 µg/jour, éventuellement où le au moins un agoniste β-adrénergique comprend du formotérol et la quantité thérapeutiquement efficace de formotérol est d'environ 0,001 µg/jour à environ 50 µ/jour.

12. Quantités thérapeutiquement efficaces des agents pour l'utilisation selon l'une quelconque des revendications 1 à 11, où le au moins un glucocorticostéroïde comprend la dexaméthasone, la prednisolone, la méthylprednisolone, la fluticasone, le budésonide, ou l'une quelconque de leurs combinaisons.

13. Quantités thérapeutiquement efficaces des agents pour l'utilisation selon l'une quelconque des revendications 1 à 12, comprenant en outre un agent immunodépresseur, lequel agent immunodépresseur est administré par voie intraoculaire, intra-orbitaire, ophtalmique, périorbitaire, rétrobulbaire, ou intra-conique avant l'administration d'au moins un composé, éventuellement où l'agent immunodépresseur est présent en une quantité thérapeutiquement efficace qui est administrée sous la forme d'une suspension de microparticules cristallines.

14. Quantités thérapeutiquement efficaces des agents pour l'utilisation selon l'une quelconque des revendications 1 à 13, où l'utilisation sensibilise l'accumulation de graisse orbitaire.

15. Préparation comprenant les quantités thérapeutiquement efficaces des agents pour l'utilisation selon l'une quelconque des revendications 1 à 14, où la composition comprend un mélange d'au moins un agoniste β-adrénergique à action prolongée et au moins un agoniste β-adrénergique à action rapide ; et éventuellement la composition comprend en outre une quantité thérapeutiquement efficace de hyaluronidase.

16. Préparation comprenant les quantités thérapeutiquement efficaces des agents pour l'utilisation selon l'une quelconque des revendications 1 à 14, où le au moins un agoniste β-adrénergique comprend le salmétérol, le formotérol, le bambutérol, le eformotérol, l'isoprotérénol, l'albutérol ou le fénotérol, où éventuellement soit (i) la composition comprend du salmétérol et la quantité thérapeutiquement efficace de salmétérol est d'environ 0,01 µg/jour à environ 100 µg/jour ; soit (ii) la composition comprend du formotérol et la quantité thérapeutiquement efficace de formotérol est d'environ 0,001 µg/jour à environ 50 µg/jour ; soit (iii) l'administration de la composition s'effectue par voie parentérale, orale, intraoculaire, intra-orbitaire, intra-conique, ophtalmique, rétrobulbaire, topique, intramusculaire, transdermique, sublinguale, intranasale ou respiratoire.

17. Quantités thérapeutiquement efficaces des agents pour l'utilisation selon l'une quelconque des revendications 1 à 16, où le patient souffre d'une maladie impliquant une accumulation de graisse orbitaire.

18. Quantités thérapeutiquement efficaces des agents pour l'utilisation selon l'une quelconque des revendications 1 à 17, où la préparation comprend du xinafoate de salmétérol.

19. Quantités thérapeutiquement efficaces des agents pour l'utilisation selon l'une quelconque des revendications 1 à 18, où l'agoniste de récepteur β-adrénergique est le fumarate de formotérol.

20. Quantités thérapeutiquement efficaces des agents pour l'utilisation selon les revendications 1 à 19, où le glucocorticostéroïde est le propionate de fluticasone.

21. Quantités thérapeutiquement efficaces des agents pour l'utilisation selon les revendications 1 à 20, où ledit au moins un agoniste de récepteur β-adrénergique et ledit au moins un glucocorticostéroïde sont formulés pour être administrés audit patient en une quantité suffisante pour parvenir à une réduction de l'accumulation de graisse orbitaire.

22. Quantités thérapeutiquement efficaces des agents pour l'utilisation selon l'une quelconque des revendications 1 à 20, où ledit au moins un agoniste de récepteur β-adrénergique et ledit au moins un glucocorticostéroïde sont formulés sous la forme d'une préparation combinée.
